(19) Europäisches Patentamt / European Patent Office / Office européen des brevets

(11) **EP 4 332 601 B1**

(12) **EUROPÄISCHE PATENTSCHRIFT**

(45) Veröffentlichungstag und Bekanntmachung des Hinweises auf die Patenterteilung:
**18.12.2024 Patentblatt 2024/51**

(21) Anmeldenummer: **22193834.3**

(22) Anmeldetag: **05.09.2022**

(51) Internationale Patentklassifikation (IPC):
**G01R 33/56** (2006.01)    **A61B 6/00** (2024.01)
**A61B 8/00** (2006.01)

(52) Gemeinsame Patentklassifikation (CPC):
**A61B 6/481; A61B 8/481; G01R 33/5601;
G01R 33/5608**

(54) **ERZEUGUNG VON KÜNSTLICHEN KONTRASTMITTELVERSTÄRKTEN RADIOLOGISCHEN AUFNAHMEN**

GENERATION OF ARTIFICIAL CONTRAST AGENT-ENHANCED RADIOLOGICAL RECORDINGS

GÉNÉRATION D'ENREGISTREMENTS RADIOLOGIQUES ARTIFICIELS RENFORCÉS PAR UN AGENT DE CONTRASTE

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**

(43) Veröffentlichungstag der Anmeldung:
**06.03.2024 Patentblatt 2024/10**

(73) Patentinhaber: **Bayer AG
51373 Leverkusen (DE)**

(72) Erfinder:
- **KREIS, Felix, Karl
 51373 Leverkusen (DE)**
- **BALTRUSCHAT, Ivo, Matteo
 51373 Leverkusen (DE)**
- **LENGA, Matthias
 51373 Leverkusen (DE)**
- **JOST, Gregor
 51373 Leverkusen (DE)**
- **SCHÜTZ, Gunnar
 51373 Leverkusen (DE)**
- **KNOBLOCH, Gesine
 51373 Leverkusen (DE)**
- **LIENERTH, Christian
 51373 Leverkusen (DE)**
- **PIETSCH, Hubertus
 51373 Leverkusen (DE)**

(74) Vertreter: **BIP Patents
c/o Bayer Intellectual Property GmbH
Alfred-Nobel-Straße 50
40789 Monheim am Rhein (DE)**

(56) Entgegenhaltungen:
WO-A1-2014/033163    WO-A1-2019/074938
WO-A1-2021/030157    US-A- 6 039 931

EP 4 332 601 B1

**Beschreibung**

Die vorliegende Offenbarung befasst sich mit dem technischen Gebiet der Erzeugung von künstlichen kontrastmittelverstärkten radiologischen Aufnahmen.

WO2019/074938A1 offenbart ein Verfahren zur Reduzierung der Menge an Kontrastmittel bei der Erzeugung von radiologischen Aufnahmen mit Hilfe eines künstlichen neuronalen Netzes.

In dem offenbarten Verfahren wird in einem ersten Schritt ein Trainingsdatensatz erzeugt. Der Trainingsdatensatz umfasst für eine Vielzahl an Personen für jede Person i) eine native radiologische Aufnahme (*zero-contrast image*), ii) eine radiologische Aufnahme nach der Applikation einer geringen Menge an Kontrastmittel (*low-contrast image*) und iii) eine radiologische Aufnahme nach der Applikation einer Standardmenge an Kontrastmittel (*full-contrast image*).

In einem zweiten Schritt wird ein künstliches neuronales Netz trainiert, für jede Person des Trainingsdatensatzes auf Basis der nativen Aufnahme und der Aufnahme nach Applikation einer geringen Menge an Kontrastmittel eine künstliche radiologische Aufnahme vorherzusagen, die einen Aufnahmebereich nach der Applikation der Standardmenge an Kontrastmittel zeigt. Die gemessene radiologische Aufnahme nach der Applikation einer Standardmenge an Kontrastmittel dient beim Training jeweils als Referenz (*ground truth*).

In einem dritten Schritt kann das trainierte künstliche neuronale Netz verwendet werden, für eine neue Person auf Basis einer nativen Aufnahme und einer radiologischen Aufnahme nach der Applikation einer geringen Menge an Kontrastmittel eine künstliche radiologische Aufnahme vorherzusagen, die den aufgenommenen Bereich so zeigt wie er aussehen würde, wenn eine Standardmenge an Kontrastmittel appliziert worden wäre.

Das in WO2019/074938A1 offenbarte Verfahren weist Nachteile auf.

So sind für das Trainieren des künstlichen neuronalen Netzes Trainingsdaten erforderlich. Es müssen eine Vielzahl an radiologischen Untersuchungen an einer Vielzahl an Personen vorgenommen und die Trainingsdaten generiert werden, um das Netz trainieren zu können.

Das in WO2019/074938A1 offenbarte künstliche neuronale Netz ist trainiert, eine radiologische Aufnahme nach der Applikation einer Standardmenge eines Kontrastmittels vorherzusagen. Das künstliche neuronale Netz ist nicht konfiguriert und nicht trainiert, eine radiologische Aufnahme nach der Applikation einer geringeren oder höheren Menge als der Standarmenge an Kontrastmittel vorherzusagen. Das in WO2019/074938A1 beschriebene Verfahren kann prinzipiell trainiert werden, eine radiologische Aufnahme nach der Applikation einer anderen Menge als der Standardmenge an Kontrastmittel vorherzusagen. Allerdings sind hierfür weitere Trainingsdaten und ein weiteres Training erforderlich.

WO2014/033163A1 offenbart ein Verfahren und eine Vorrichtung zur Ermittlung eines Wertes einer relativen Kontrastmittelanreicherung bei einer kontrastmittelverstärkten Magnetresonanzaufnahme (MRT) basierend auf einer Untermenge von Daten aus einem ersten Datensatz und basierend auf einer Untermenge von Daten aus einem zweiten Datensatz, wobei der erste Datensatz Daten eines mehrdimensionalen k-Raumes einer ohne Einfluss eines Kontrastmittels aufgenommenen Magnetresonanzaufnahme umfasst und in einem ersten Zeitabschnitt aufgenommen worden ist, und wobei der zweite Datensatz Daten eines mehrdimensionalen k-Raumes einer mit Einfluss eines Kontrastmittels aufgenommenen Magnetresonanzaufnahme umfasst und in dem zweiten Zeitabschnitt, welcher unterschiedlich zum ersten Zeitabschnitt ist, aufgenommen worden ist.

Es wäre wünschenswert, radiologische Aufnahmen mit einer variablen Kontrastverstärkung erzeugen zu können, ohne dass für die einzelne Kontrastverstärkung Trainingsdaten erzeugt und eine künstliches neuronales Netz trainiert werden müssen. Es wäre weiterhin wünschenswert radiologische Aufnahmen mit einer variablen Kontrastverstärkung erzeugen zu können, wobei ein nachvollziehbarer, deterministischer Prozess zur Erzeugung der variablen Kontrastverstärkung angewendet wird. Dies erleichtert Zulassung und Anwendung eines entsprechenden Verfahrens im medizinischen Bereich, in dem falsch negative und falsch positive Befunde zu minimieren sind. Methoden des maschinellen Lernens nutzen statistische Modelle, deren Generalisierbarkeit beschränkt ist, da ihnen üblicherweise eine beschränkte Auswahl an Trainingsdaten zu Grunde liegt.

Diese und weitere Aufgaben werden durch die Gegenstände der unabhängigen Patentansprüche gelöst. Bevorzugte Ausführungsformen der vorliegenden Offenbarung finden sich in den abhängigen Patentansprüchen, in der vorliegenden Beschreibung und in den Zeichnungen.

Ein erster Gegenstand der vorliegenden Offenbarung ist somit ein computer-implementiertes Verfahren zur Erzeugung einer kontrastverstärkten radiologischen Aufnahme, umfassend die Schritte:

- Empfangen oder Erzeugen einer ersten Repräsentation, wobei die erste Repräsentation einen Untersuchungsbereich eines Untersuchungsobjekts ohne Kontrastmittel oder nach der Applikation einer ersten Menge eines Kontrastmittels im Frequenzraum repräsentiert,

- Empfangen oder Erzeugen einer zweiten Repräsentation, wobei die zweite Repräsentation den Untersuchungsbereich des Untersuchungsobjekts nach einer Applikation einer zweiten Menge des Kontrastmittels im Frequenzraum repräsentiert,

- Erzeugen einer dritten Repräsentation auf Basis der ersten Repräsentation und der zweiten Repräsentation, wobei die dritte Repräsentation die im Untersuchungsbereich durch die zweite Menge an Kontrastmittel hervorgerufene Signalverstärkung im Frequenzraum repräsentiert,

- Erzeugen einer gewichteten dritten Repräsentation durch Anwenden einer frequenzabhängigen Gewichtsfunktion auf die dritte Repräsentation,

- Erzeugen einer vierten Repräsentation durch Kombinieren der ersten Repräsentation mit der gewichteten dritten Repräsentation,

- Transformieren der vierten Repräsentation in eine Repräsentation des Untersuchungsbereichs im Ortsraum,

- Ausgeben und/oder Speichern der Repräsentation des Untersuchungsbereichs im Ortsraum und/oder Übermitteln der Repräsentation des Untersuchungsbereichs im Ortsraum an ein separates Computersystem.

Ein weiterer Gegenstand der vorliegenden Offenbarung ist ein Computersystem umfassend

• eine Empfangseinheit,

• eine Steuer- und Recheneinheit und

• eine Ausgabeeinheit,

wobei die Steuer- und Recheneinheit konfiguriert ist,

- die Empfangseinheit zu veranlassen, eine erste Repräsentation zu empfangen, wobei die erste Repräsentation einen Untersuchungsbereich eines Untersuchungsobjekts ohne Kontrastmittel oder nach der Applikation einer ersten Menge eines Kontrastmittels im Frequenzraum repräsentiert, oder eine erste Ortsraum-Repräsentation zu empfangen, wobei die erste Ortsraum-Repräsentation den Untersuchungsbereich des Untersuchungsobjekts ohne Kontrastmittel oder nach der Applikation der ersten Menge des Kontrastmittels im Ortsraum repräsentiert,

- für den Fall, dass von der Empfangseinheit die erste Ortsraum-Repräsentation empfangen wurde: die erste Ortsraum-Repräsentation in die erste Repräsentation des Untersuchungsbereich des Untersuchungsobjekts im Frequenzraum zu transformieren,

- die Empfangseinheit zu veranlassen, eine zweite Repräsentation zu empfangen, wobei die zweite Repräsentation den Untersuchungsbereich des Untersuchungsobjekts nach einer Applikation einer zweiten Menge des Kontrastmittels im Frequenzraum repräsentiert, oder eine zweite Ortsraum-Repräsentation zu empfangen, wobei die zweite Ortsraum-Repräsentation den Untersuchungsbereich des Untersuchungsobjekts nach der Applikation der zweiten Menge des Kontrastmittels im Ortsraum repräsentiert,

- für den Fall, dass von der Empfangseinheit die zweite Ortsraum-Repräsentation empfangen wurde: die zweite Ortsraum-Repräsentation in die zweite Repräsentation des Untersuchungsbereich des Untersuchungsobjekts im Frequenzraum zu transformieren,

- eine dritte Repräsentation auf Basis der ersten Repräsentation und der zweiten Repräsentation zu erzeugen, wobei die dritte Repräsentation die im Untersuchungsbereich durch die zweite Menge an Kontrastmittel hervorgerufene Signalverstärkung im Frequenzraum repräsentiert,

- eine gewichtete dritte Repräsentation durch Anwenden einer frequenzabhängigen Gewichtsfunktion auf die dritte Repräsentation zu erzeugen,

- eine vierte Repräsentation durch Kombinieren der ersten Repräsentation mit der gewichteten dritten Repräsentation zu erzeugen,

- die vierte Repräsentation in eine Repräsentation des Untersuchungsbereichs im Ortsraum zu transformieren,

- die Ausgabeeinheit zu veranlassen, die Repräsentation des Untersuchungsbereichs im Ortsraum auszugeben und/oder zu speichern und/oder an ein separates Computersystem zu übermitteln.

Ein weiterer Gegenstand der vorliegenden Offenbarung ist ein Computerprogramm, das in einen Arbeitsspeicher eines Computersystems geladen werden kann und dort das Computersystem dazu veranlasst, folgende Schritte ausführen:

- Empfangen oder Erzeugen einer ersten Repräsentation, wobei die erste Repräsentation einen Untersuchungsbereich eines Untersuchungsobjekts ohne Kontrastmittel oder nach der Applikation einer ersten Menge eines Kontrastmittels im Frequenzraum repräsentiert,

- Empfangen oder Erzeugen einer zweiten Repräsentation, wobei die zweite Repräsentation den Untersuchungsbereich des Untersuchungsobjekts nach einer Applikation einer zweiten Menge des Kontrastmittels im Frequenzraum repräsentiert,

- Erzeugen einer dritten Repräsentation auf Basis der ersten Repräsentation und der zweiten Repräsentation, wobei die dritte Repräsentation die im Untersuchungsbereich durch die zweite Menge an Kontrastmittel hervorgerufene Signalverstärkung im Frequenzraum repräsentiert,

- Erzeugen einer gewichteten dritten Repräsentation durch Anwenden einer frequenzabhängigen Gewichtsfunktion auf die dritte Repräsentation,

- Erzeugen einer vierten Repräsentation durch Kombinieren der ersten Repräsentation mit der gewichteten dritten Repräsentation,

- Transformieren der vierten Repräsentation in eine Repräsentation des Untersuchungsbereichs im Ortsraum,

- Ausgeben und/oder Speichern der Repräsentation des Untersuchungsbereichs im Ortsraum und/oder Übermitteln der Repräsentation des Untersuchungsbereichs im Ortsraum an ein separates Computersystem.

Ein weiterer Gegenstand der vorliegenden Offenbarung ist ein Kit umfassend ein Computerprogrammprodukt und ein Kontrastmittel, wobei das Computerprogrammprodukt ein Computerprogramm umfasst, das in einen Arbeitsspeicher eines Computersystems geladen werden kann und dort das Computersystem dazu veranlasst, folgende Schritte ausführen:

- Empfangen einer ersten Repräsentation, wobei die erste Repräsentation einen Untersuchungsbereich eines Untersuchungsobjekts ohne das Kontrastmittel oder nach der Applikation einer ersten Menge des Kontrastmittels im Frequenzraum repräsentiert,

- Empfangen einer zweiten Repräsentation, wobei die zweite Repräsentation den Untersuchungsbereich des Untersuchungsobjekts nach einer Applikation einer zweiten Menge des Kontrastmittels im Frequenzraum repräsentiert,

- Erzeugen einer dritten Repräsentation auf Basis der ersten Repräsentation und der zweiten Repräsentation, wobei die dritte Repräsentation die im Untersuchungsbereich durch die zweite Menge an Kontrastmittel hervorgerufene Signalverstärkung im Frequenzraum repräsentiert,

- Erzeugen einer gewichteten dritten Repräsentation durch Anwenden einer frequenzabhängigen Gewichtsfunktion auf die dritte Repräsentation,

- Erzeugen einer vierten Repräsentation durch Kombinieren der ersten Repräsentation mit der gewichteten dritten Repräsentation,

- Transformieren der vierten Repräsentation in eine Repräsentation des Untersuchungsbereichs im Ortsraum,

- Ausgeben und/oder Speichern der Repräsentation des Untersuchungsbereichs im Ortsraum und/oder Übermitteln der Repräsentation des Untersuchungsbereichs im Ortsraum an ein separates Computersystem.

Die Gegenstände der vorliegenden Offenbarung werden nachstehend näher erläutert, ohne zwischen den Gegenständen (Verfahren, Computersystem, Computerprogramm(produkt), Kit) zu unterscheiden. Die nachfolgenden Erläuterungen sollen vielmehr für alle Gegenstände in analoger Weise gelten, unabhängig davon, in welchem Kontext (Verfahren, Computersystem, Computerprogramm(produkt), Kit) sie erfolgen.

Wenn in der vorliegenden Beschreibung oder in den Patentansprüchen Schritte in einer Reihenfolge genannt sind, bedeutet dies nicht zwingend, dass diese Offenbarung auf die genannte Reihenfolge beschränkt ist. Vielmehr ist denkbar, dass die Schritte auch in einer anderen Reihenfolge oder auch parallel zueinander ausgeführt werden; es sei denn, ein Schritt baut auf einem anderen Schritt auf, was zwingend erforderlich macht, dass der aufbauende Schritt nachfolgend ausgeführt wird (was im Einzelfall aber deutlich wird). Die genannten Reihenfolgen stellen damit bevorzugte Ausführungsformen dar.

Die vorliegende Offenbarung beschreibt Mittel, mit denen auf Basis von mindestens zwei Repräsentationen, die einen Untersuchungsbereich eines Untersuchungsobjekts mit unterschiedlichen Mengen an Kontrastmittel repräsentieren, eine oder mehrere künstliche radiologische Aufnahmen erzeugt werden, bei der/denen der Kontrast zwischen Bereichen mit Kontrastmittel und Bereichen ohne Kontrastmittel variiert werden kann.

Das "Untersuchungsobjekt" ist üblicherweise ein Lebewesen, vorzugsweise ein Säugetier, ganz besonders bevorzugt ein Mensch.

Der "Untersuchungsbereich" ist ein Teil des Untersuchungsobjekts, zum Beispiel ein Organ oder ein Teil eines Organs.

Der Untersuchungsbereich kann beispielsweise eine Leber, eine Niere, ein Herz, eine Lunge, ein Gehirn, ein Magen, eine Blase, eine Prostatadrüse, ein Darm oder ein Teil davon oder ein anderer Teil des Körpers eines Menschen sein. In einer bevorzugten Ausführungsform umfasst der Untersuchungsbereich die Leber oder einen Teil der Leber oder der Untersuchungsbereich ist die Leber oder ein Teil der Leber.

Der Untersuchungsbereich, auch Aufnahmevolumen (engl.: *field of view*, FOV) genannt, stellt insbesondere ein Volumen dar, welches in radiologischen Aufnahmen abgebildet wird. Der Untersuchungsbereich wird typischerweise durch einen Radiologen, beispielsweise auf einer Übersichtsaufnahme (engl.: *localizer*) festgelegt. Selbstverständlich kann der Untersuchungsbereich alternativ oder zusätzlich auch automatisch, beispielsweise auf Grundlage eines ausgewählten Protokolls, festgelegt werden.

Der Untersuchungsbereich wird einer radiologischen Untersuchung unterzogen.

Die "Radiologie" ist das Teilgebiet der Medizin, das sich mit der Anwendung elektromagnetischer Strahlen und (unter Einbezug etwa der Ultraschalldiagnostik) mechanischer Wellen zu diagnostischen, therapeutischen und/oder wissenschaftlichen Zwecken befasst. Neben Röntgenstrahlen kommen auch andere ionisierende Strahlung wie Gammastrahlung oder Elektronen zum Einsatz. Da ein wesentlicher Einsatzzweck die Bildgebung ist, werden auch andere bildgebende Verfahren wie die Sonografie und die Magnetresonanztomographie (Kernspintomographie) zur Radiologie gerechnet, obwohl bei diesen Verfahren keine ionisierende Strahlung zum Einsatz kommt. Der Begriff "Radiologie" im Sinne der vorliegenden Erfindung umfasst damit insbesondere die folgenden Untersuchungsmethoden: Computertomografie, Magnetresonanztomografie, Sonografie.

In einer bevorzugten Ausführungsform der vorliegenden Erfindung handelt es sich bei der radiologischen Untersuchung um eine magnetresonanztomographische Untersuchung oder eine computertomografische Untersuchung.

Bei radiologischen Untersuchungen werden häufig Kontrastmittel zur Kontrastverstärkung eingesetzt. "Kontrastmittel" sind Substanzen oder Gemische von Substanzen, die die Darstellung von Strukturen und Funktionen des Körpers bei radiologischen Untersuchungen verbessern.

In der Computertomographie werden meist iodhaltige Lösungen als Kontrastmittel eingesetzt. In der Magnetresonanztomographie (MRT) werden üblicherweise superparamagnetische Substanzen (z.B. Eisenoxidnanopartikel, superparamagnetische Eisen-Platin-Partikel (SIPPs)) oder paramagnetische Substanzen (z.B. Gadolinium-Chelate, Mangan-Chelate) als Kontrastmittel verwendet. Im Fall der Sonografie werden üblicherweise Flüssigkeiten, die gasgefüllte Mikrobläschen (*microbubbles*) enthalten, intravenös verabreicht. Beispiele für Kontrastmittel sind in der Literatur zu finden (siehe z.B. A. S. L. Jascinth et al.: Contrast Agents in computed tomography: A Review, Journal of Applied Dental and Medical Sciences, 2016, Vol. 2, Issue 2, 143 - 149; H. Lusic et al.: X-ray-Computed Tomography Contrast Agents, Chem. Rev. 2013, 113, 3, 1641-1666; https://www.radiology.wisc.edu/wpcontent/uploads/2017/10/contrast-agents-tutorial.pdf, M. R. Nough et al.: Radiographie and magnetic resonances contrast agents: Essentials and tips for safe practices, World J Radiol. 2017 Sep 28; 9(9): 339-349; L. C. Abonyi et al.: Intravascular Contrast Media in Radiography: Historical Development & Review ofRisk Factors for Adverse Reactions, South American Journal of Clinical Research, 2016, Vol. 3, Issue 1, 1-10; ACR Manual on Contrast Media, 2020, ISBN: 978-1-55903-012-0; A. Ignee et al.: Ultrasound contrast agents, Endosc Ultrasound. 2016 Nov-Dec; 5(6): 355-362).

MR-Kontrastmittel entfalten ihre Wirkung, indem sie die Relaxationszeiten der Strukturen, die Kontrastmittel aufnehmen, verändern. Es lassen sich zwei Stoffgruppen unterscheiden: para- und superparamagnetische Stoffe. Beide Stoffgruppen besitzen ungepaarte Elektronen, die ein magnetisches Feld um die einzelnen Atome bzw. Moleküle induzieren. Superparamagnetische führen zu einer überwiegenden T2-Verkürzung, während paramagnetische Kontrastmittel im Wesentlichen zu einer T1-Verkürzung führen. Die Wirkung dieser Kontrastmittel ist indirekt, da das Kontrastmittel selbst kein Signal abgibt, sondern nur die Signalintensität in seiner Umgebung beeinflusst. Ein Beispiel für ein superparamagnetisches Kontrastmittel sind Eisenoxidnanopartikel (SPIO, engl.: *superparamagnetic iron oxide*). Beispiele für paramagnetische Kontrastmittel sind Gadolinium-Chelate wie Gadopentetat-Dimeglumin (Handelsname: Magnevist® u.a.), Gadotersäure (Dotarem®, Dotagita®, Cyclolux®), Gadodiamid (Omniscan®), Gadoteridol (ProHance®), Gadobutrol (Gadovist®) und Gadoxetsäure (Primovist®/Eovist®).

Die Erzeugung einer künstlichen radiologischen Aufnahme mit einer variablen Kontrastverstärkung basiert auf mindestens zwei Repräsentationen des Untersuchungsbereichs, einer ersten Repräsentation und einer zweiten Repräsentation.

Die erste Repräsentation repräsentiert den Untersuchungsbereich ohne Kontrastmittel oder nach der Applikation einer ersten Menge eines Kontrastmittels. Vorzugsweise repräsentiert die erste Repräsentation den Untersuchungsbereich ohne Kontrastmittel.

Die zweite Repräsentation repräsentiert den Untersuchungsbereich nach der Applikation einer zweiten Menge eines Kontrastmittels. Die zweite Menge ist größer als die erste Menge (wobei die erste Menge wie beschrieben auch Null sein kann). Der Ausdruck "nach der zweiten Menge eines Kontrastmittels" soll nicht so verstanden werden, dass sich die erste Menge und die zweite Menge in dem Untersuchungsbereich addieren (es sei denn die erste Menge ist Null). Der Ausdruck "die Repräsentation repräsentiert den Untersuchungsbereich nach der Applikation einer (ersten oder zweiten) Menge" soll also eher bedeuten: "die Repräsentation repräsentiert den Untersuchungsbereich mit einer (ersten oder zweiten) Menge" oder "die Repräsentation repräsentiert den Untersuchungsbereich umfassend eine (erste oder zweite) Menge".

Vorzugsweise ist sowohl die erste Menge als auch die zweite Menge des Kontrastmittels kleiner als die Standardmenge.

Die Standardmenge ist üblicherweise die vom Hersteller und/oder Vertreiber des Kontrastmittels empfohlene und/oder die von einer Zulassungsbehörde zugelassene und/oder die in einem Beipackzettel zum Kontrastmittel aufgeführte Menge.

So beträgt die Standardmenge von Primovist® beispielsweise 0,025 mmol Gd-EOB-DTPA Dinatrium / kg Körpergewicht.

Die erste Repräsentation und die zweite Repräsentation repräsentieren den Untersuchungsbereich im Frequenzraum.

Die Rohdaten, die bei magnetresonanztomografischen Untersuchungen anfallen (so genannte k-Raum-Daten) sind ein Beispiel für eine Repräsentation im Frequenzraum. Solche Rohdaten (k-Raum-Daten) aus magnetresonanztomografischen Untersuchungen können direkt als erste und/oder zweite Repräsentation im Sinne der vorliegenden Offenbarung verwendet werden.

Üblicherweise fallen radiologische Aufnahmen als Ergebnis von radiologischen Untersuchungen (insbesondere im Fall der Computertomografie und der Sonografie) als Repräsentationen im Ortsraum (auch Bildraum genannt) an. Diese Repräsentationen sind die für Menschen geläufigere Repräsentation. In einer Repräsentation im Ortsraum, in dieser Beschreibung auch als Ortsraumdarstellung oder Ortsraum-Repräsentation bezeichnet, wird der Untersuchungsbereich üblicherweise durch eine Vielzahl an Bildelementen (Pixel oder Voxel) repräsentiert, die beispielsweise rasterförmig angeordnet sein können, wobei jedes Bildelement einen Teil des Untersuchungsbereichs repräsentiert, wobei jedem Bildelement ein Farbwert oder Grauwert zugeordnet sein kann. Ein in der Radiologie weit verbreitetes Format zur Speicherung und Verarbeitung von Repräsentationen im Ortsraum ist das DICOM-Format. DICOM (Digital Imaging and Communications in Medicine) ist ein offener Standard zur Speicherung und zum Austausch von Informationen im medizinischen Bilddatenmanagement.

Eine Repräsentation im Ortsraum lässt sich beispielsweise durch eine Fourier-Transformation in eine Repräsentation im Frequenzraum überführen (transformieren). Umgekehrt lässt sich eine Repräsentation im Frequenzraum beispielsweise durch eine inverse Fourier-Transformation in eine Repräsentation im Ortsraum überführen (transformieren).

In einer Repräsentation im Frequenzraum, in dieser Beschreibung auch als Frequenzraumdarstellung oder Frequenzraum-Repräsentation bezeichnet, wird der Untersuchungsbereich durch eine Überlagerung von Grundschwingungen repräsentiert. Beispielsweise kann der Untersuchungsbereich durch eine Summe von Sinus- und/oder Kosinus-Funktionen mit verschiedenen Amplituden, Frequenzen und Phasen repräsentiert werden. Die Amplituden und Phasen können als Funktion der Frequenzen beispielsweise in einer zwei- oder dreidimensionalen Darstellung aufgetragen werden. Üblicherweise wird die niedrigste Frequenz (Ursprung) in das Zentrum gelegt. Je weiter man sich von diesem Zentrum entfernt, desto höher sind die Frequenzen. Jeder Frequenz kann eine Amplitude, mit der die Frequenz in der Frequenzraumdarstellung vertreten ist, und eine Phase, die angibt, inwieweit die jeweilige Schwingung gegenüber einer Sinus- oder Kosinus-Schwingung verschoben ist, zugeordnet werden.

Details über Ortsraumdarstellungen und Frequenzraumdarstellungen und ihre jeweilige Umwandlung ineinander sind in zahlreichen Publikationen beschrieben, siehe z.B.: https://see.stanford.edu/materials/lsoftaee261/book-fall-07.pdf.

Wenn also die erste Repräsentation und/oder die zweite Repräsentation den Untersuchungsbereich im Ortsraum repräsentieren, dann wird die jeweilige Ortsraum-Repräsentation zunächst durch eine Transformation (z.B. eine Fourier-Transformation) in eine Repräsentation im Frequenzraum überführt (umgewandelt, transformiert).

Es ist möglich, eine Co-Registrierung von Repräsentationen im Ortsraum durchzuführen, bevor sie in die Frequenzraumdarstellungen umgewandelt werden. Die "Co-Registrierung" (im Stand der Technik auch "Bildregistrierung" genannt) dient dazu, zwei oder mehrere Ortsraumdarstellungen desselben Untersuchungsbereichs bestmöglich in Übereinstimmung miteinander zu bringen. Dabei wird eine der Ortsraumdarstellungen als Referenzbild festgelegt, die andere wird Objektbild genannt. Um diese optimal an das Referenzbild anzupassen, wird eine ausgleichende Transformation berechnet.

Es ist auch möglich, eine Co-Registrierung von Repräsentationen im Frequenzraum durchzuführen, wobei hierbei zu beachten ist, dass sich eine Translation im Ortsraum als eine additive lineare Phasenrampe im Frequenzraum darstellt. Skalierung und Rotation hingegen bleiben bei der Fourier- und inversen Fourier-Transformation erhalten - eine Skalierung und Rotation im Frequenzraum ist auch eine Skalierung und Rotation im Ortsraum (siehe z.B.: S. Skare: Rigid Body Image Realignment in Image Space vs. k-Space, ISMRM SCIENTIFIC WORKSHOP on Motion Correction, 2014, https://cds.ismrm.org/protected/Motion_14/Program/Syllabus/Skare.pdf).

Allerdings sei angemerkt, dass die Co-Registrierung im Frequenzraum nicht sehr präzise sein muss, da hohe Frequenzen, welche Bilddetails und somit Ungenauigkeiten in der Registrierung abbilden durch den Frequenzfilter abgeschwächt werden. Dies ist ein Vorteil des in dieser Offenbarung beschriebenen Ansatzes gegenüber Ansätzen, bei denen Operationen im Ortsraum ausgeführt werden.

Auf Basis der ersten Repräsentation und der zweiten Repräsentation wird eine dritte Repräsentation erzeugt.

Die dritte Repräsentation repräsentiert zumindest einen Teil des Untersuchungsbereichs im Frequenzraum.

Die dritte Repräsentation repräsentiert die durch die zweite Menge an Kontrastmittel im Untersuchungsbereich hervorgerufene Signalverstärkung. Mit anderen Worten, die dritte Repräsentation umfasst die Unterschiede in der zweiten Repräsentation gegenüber der ersten Repräsentation, die durch die zweite Menge an Kontrastmittel hervorgerufen werden.

In einer bevorzugten Ausführungsform wird die dritte Repräsentation durch Subtraktion der ersten Repräsentation von der zweiten Repräsentation erzeugt. Mit anderen Worten: in einer bevorzugten Ausführungsform ist die dritte Repräsentation die Differenz der ersten und der zweiten Repräsentation. In der dritten Repräsentation ist jede Frequenz mit einem umso höheren Amplitudenwert vertreten, je stärker die Frequenz von der zweiten Menge an Kontrastmittel beeinflusst wird.

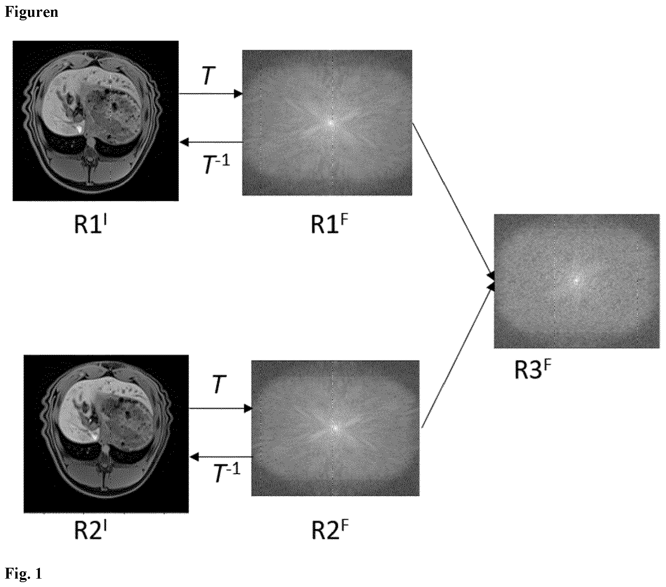

Fig. 1 zeigt die Erzeugung einer dritten Repräsentation auf Basis einer ersten Repräsentation und einer zweiten Repräsentation schematisch in Form eines Beispiels.

Fig. 1 zeigt einen Untersuchungsbereich eines Untersuchungsobjekts in Form von verschiedenen Repräsentation.

Eine erste Repräsentation R1$^I$ repräsentiert den Untersuchungsbereich im Ortsraum ohne Kontrastmittel oder nach der Applikation einer ersten Menge eines Kontrastmittels. Der in Fig. 1 gezeigte Untersuchungsbereich umfasst eine Leber eines Schweines. Die erste Repräsentation R1$^I$ ist eine magnetresonanztomografische Aufnahme.

Die erste Ortsraum-Repräsentation R1$^I$ lässt sich durch eine Transformation T, zum Beispiel einer Fourier-Transformation, in eine erste Repräsentation R1$^F$ des Untersuchungsbereich im Frequenzraum umwandeln. Die erste Frequenzraum-Repräsentation R1$^F$ repräsentiert denselben Untersuchungsbereich desselben Untersuchungsobjekts wie die erste Ortsraum-Repräsentation R1$^I$, ebenfalls ohne Kontrastmittel oder nach der Applikation der ersten Menge des Kontrastmittels.

Die erste Frequenzraum-Repräsentation R1$^F$ lässt sich mittels einer inversen Transformation $T^{-1}$ in die erste Ortsraum-Repräsentation R1$^I$ umwandeln. Die inverse Transformation $T^{-1}$ ist die zur Transformation T inverse Transformation.

Eine zweite Repräsentation R2$^I$ repräsentiert denselben Untersuchungsbereich desselben Untersuchungsobjekts wie die erste Repräsentation R1$^I$ im Ortsraum. Die zweite Ortsraum-Repräsentation R2$^I$ repräsentiert den Untersuchungsbereich nach der Applikation einer zweiten Menge des Kontrastmittels. Die zweite Menge ist größer als die erste Menge (wobei die erste Menge wie beschrieben auch Null sein kann). Die zweite Repräsentation R1$^I$ ist ebenfalls eine magnetresonanztomografische Aufnahme. Als Kontrastmittel wurde ein hepatobiliäres Kontrastmittel verwendet. Ein hepatobiliäres Kontrastmittel zeichnet sich dadurch aus, dass es spezifisch von Leberzellen, den Hepatozyten, aufgenommen wird, sich im Funktionsgewebe (Parenchym) anreichert und den Kontrast in gesundem Lebergewebe verstärkt. Ein Beispiel eines hepatobiliären Kontrastmittels ist das Dinatriumsalz der Gadoxetsäure (Gd-EOB-DTPA Dinatrium), das in dem US-Patent No. 6,039,931A beschrieben ist unter dem Markennamen Primovist® und Eovist® kommerziell erhältlich ist. In der zweiten Ortsraum-Repräsentation R2$^I$ ist der Kontrast zwischen dem Lebergewebe und dem umliegenden Gewebe infolge der zweiten Menge des Kontrastmittels erhöht.

Die zweite Ortsraum-Repräsentation R2$^I$ lässt sich durch die Transformation T in eine zweite Repräsentation R2$^F$ des Untersuchungsbereich im Frequenzraum umwandeln. Die zweite Frequenzraum-Repräsentation R2$^F$ repräsentiert denselben Untersuchungsbereich desselben Untersuchungsobjekts wie die zweite Ortsraum-Repräsentation R2$^I$, ebenfalls nach der Applikation der zweiten Menge des Kontrastmittels.

Die zweite Frequenzraum-Repräsentation R2$^F$ lässt sich mittels der inversen Transformation $T^{-1}$ in die zweite Ortsraum-Repräsentation R2$^I$ umwandeln.

Auf Basis der ersten Frequenzraum-Repräsentation R1$^F$ und der zweiten Frequenzraum-Repräsentation R2$^F$ wird eine dritte Frequenzraum-Repräsentation R3$^F$ erzeugt. In dem in Fig. 1 gezeigten Beispiel ist die dritte Frequenzraum-Repräsentation R3$^F$ die Differenz der zweiten Frequenzraum-Repräsentation R2$^F$ und der ersten Frequenzraum-Repräsentation R1$^F$ (R3$^F$ = R2$^F$ - R1$^F$).

Die dritte Frequenzraum-Repräsentation R3$^F$ kann einer Normalisierung unterzogen werden, das heißt, die Amplitudenwerte können mit einem Faktor multipliziert werden, so dass die Amplitude mit dem höchsten Wert beispielsweise durch den Farbton weiß und die Amplitude mit dem geringsten Wert beispielsweise durch den Farbton schwarz repräsentiert wird.

Die dritte Frequenzraum-Repräsentation R3$^F$ repräsentiert die durch die zweite Menge des Kontrastmittels im Untersuchungsbereich hervorgerufene Kontrastverstärkung.

In einem weiteren Schritt wird eine gewichtete dritte Repräsentation auf Basis der dritten Repräsentation erzeugt. Durch die Gewichtung der dritten Repräsentation werden Frequenzen, die einen höheren Beitrag zum Kontrast leisten, mit einem höheren Gewicht versehen als Frequenzen, die einen geringeren Beitrag zum Kontrast leisten. Dabei bezieht sich der Ausdruck "Kontrast" auf die zur FrequenzraumDarstellung korrespondierende Ortsraum-Darstellung. Kontrastinformationen werden in einer Frequenzraumdarstellung durch niedrige Frequenzen repräsentiert, während die höheren Frequenzen Informationen zu Feinstrukturen repräsentieren. Bildrauschen ist typischerweise in der Frequenzdarstellung gleichverteilt. Die gewichtete dritte Repräsentation kann also durch Anwenden einer frequenzabhängigen Gewichtsfunktion auf die dritte Repräsentation erzeugt werden, wobei niedrige Frequenzen höher gewichtet werden als hohe Frequenzen. Die frequenzabhängige Gewichtsfunktion hat die Wirkung eines Filters. Der Filter erhöht das Signal-zu-Rauschen-Verhältnis, da die spektrale Rauschdichte für hohe Frequenzen verringert wird.

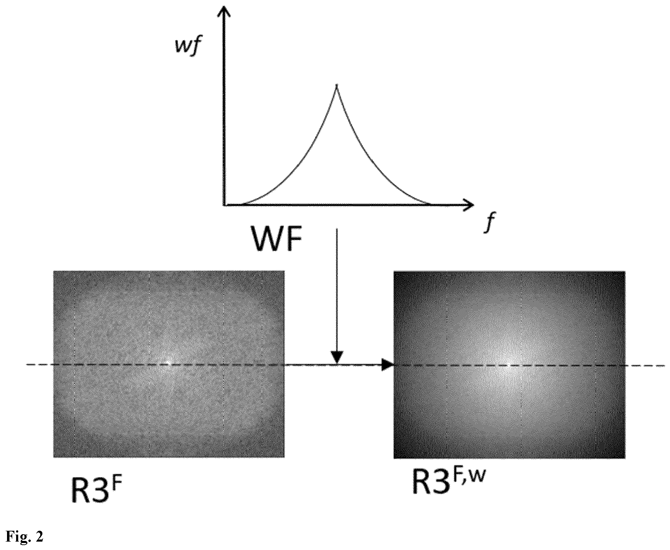

Fig. 2 zeigt beispielhaft und schematisch die Erzeugung einer gewichteten dritten Repräsentation R3$^{F,w}$. Fig. 2 zeigt die bereits in Fig. 1 gezeigte dritte Frequenzraum-Repräsentation $R3^F$. Die Amplitudenwerte der dritten Frequenzraum-Repräsentation $R3^F$ werden mit Gewichtfaktoren multipliziert. Die Gewichtfaktoren sind frequenzabhängig, d.h. die Gewichtsfaktoren sind eine Funktion der Frequenz. Aus Gründen der Veranschaulichung ist die Gewichtsfunktion WF in Fig. 2 in zweidimensionaler Form dargestellt. Die Gewichtsfunktion WF zeigt die Gewichtsfaktoren *wf* als Funktion der Frequenz f entlang einer Dimension (entlang der gestrichelten Linie). Entlang der Dimension senkrecht zu der gestrichelten Linie in derselben Bildebene hat die Gewichtsfunktion die gleiche Form; sie ist lediglich gestaucht, da die Repräsentation $R3^F$ im vorliegenden Beispiel rechteckig aber nicht quadratisch ist.

Durch die Gewichtsfunktion WF werden die Amplituden von niedrigen Frequenzen (die Frequenzen nehmen in dem gezeigten Beispiel vom Zentrum der Repräsentation $R3^F$ nach außen zu) mit einem höheren Gewichtsfaktor multipliziert als die Amplituden von höheren Frequenzen (die weiter vom Zentrum der Repräsentation $R3^F$ entfernt sind); d.h. die niedrigen Frequenzen werden höher gewichtet als die höheren Frequenzen. Dies ist in der gewichteten Repräsentation $R3^{F,w}$ daran zu erkennen, dass Grauwerte zu den Rändern der Repräsentation dunkler sind als im Fall der Repräsentation $R3^F$ und die Helligkeit insgesamt vom Zentrum nach außen schneller absinkt als bei der Repräsentation $R3^F$.

Die gewichtete Repräsentation $R3^{F,w}$ kann einer Normalisierung unterzogen werden, das heißt, die Amplitudenwerte können mit einem Faktor multipliziert werden, so dass die Amplitude mit dem höchsten Wert beispielsweise durch den Farbton weiß und die Amplitude mit dem geringsten Wert beispielsweise durch den Farbton schwarz repräsentiert wird.

Fig. 3 zeigt Beispiele für frequenzabhängige Gewichtsfunktionen, die zur Gewichtung der dritten Repräsentation verwendet werden können. Die Gewichtsfunktionen sind der Einfachheit halber als zweidimensionale Graphen dargestellt, bei denen die Gewichtsfaktoren wf (Ordinate) als Funktion der Frequenz *f* (Abszisse) aufgetragen sind.

Fig. 3 (a) zeigt die bereits in Fig. 2 gezeigte Gewichtsfunktion WF. In dieser Gewichtsfunktion können die Gewichtsfaktoren beispielsweise vom Zentrum mit zunehmender Frequenz exponentiell absinken.

Fig. 3 (b) zeigt eine Gewichtsfunktion, bei der die Gewichtsfaktoren vom Zentrum mit zunehmender Frequenz linear absinken.

Fig. 3 (c) zeigt eine Gewichtsfunktion, bei der die Gewichtsfaktoren vom Zentrum mit zunehmender Frequenz in Form einer umgekehrten Parabel absinken.

Fig. 3 (d) zeigt eine Gewichtsfunktion, bei der die Gewichtsfaktoren über einen definierten Bereich um das Zentrum herum konstant sind und dann ab einer Schwellenfrequenz exponentiell absinken.

Fig. 3 (e) zeigt eine Gewichtsfunktion, bei der die Gewichtsfaktoren in Form einer Kosinusfunktion um das Zentrum herum verlaufen.

Fig. 3 (f) zeigt eine Gewichtsfunktion, bei der die Gewichtsfaktoren in Form einer Treppenfunktion um das Zentrum herum verlaufen.

Fig. 3 (g) zeigt eine Gewichtsfunktion, bei der die Gewichtsfaktoren in Form einer Gauß-Verteilungsfunktion um das Zentrum herum verlaufen.

Fig. 3 (h) zeigt eine Gewichtsfunktion, bei der die Gewichtsfaktoren in Form einer Hann-Funktion um das Zentrum herum verlaufen.

Kombinationen der gezeigten Gewichtsfunktionen und weitere/andere Gewichtsfunktionen sind möglich. Beispiele für weitere Gewichtsfunktionen sind zum Beispiel zu finden unter https://de.wikipedia.org/wiki/Fensterfunktion#Beispiele_von_Fensterfunktionen.

Bevorzugt werden Gewichtungsfunktionen verwendet, die sich in der MR Bildgebung und Spektroskopie zur Gewichtung von k-Raum-Daten bewährt haben, wie z.B. die Hann-Funktion (*Hanning Window,* siehe z.B. R. Pohmann et al.: Accurate phosphorus metabolite images of the human heart by 3D acquisition-weighted CSI, Magnetic Resonance in Medicine: An Official Journal of the International Society for Magnetic Resonance in Medicine 45.5 (2001): 817-826).

In einem weiteren Schritt wird eine vierte Repräsentation durch Kombinieren der ersten Repräsentation mit der gewichteten dritten Repräsentation erzeugt. Durch eine solche Kombination werden Informationen zu der Kontrastverstärkung, die durch die zweite Menge des Kontrastmittels im Untersuchungsbereich hervorgerufen wird, auf die erste Repräsentation übertragen.

Die Kombination kann beispielsweise eine Addition der ersten Repräsentation und der gewichteten dritten Repräsentation sein. Es ist aber auch möglich, eine multiplikative Kombination oder eine nichtlineare Kombination durchzuführen.

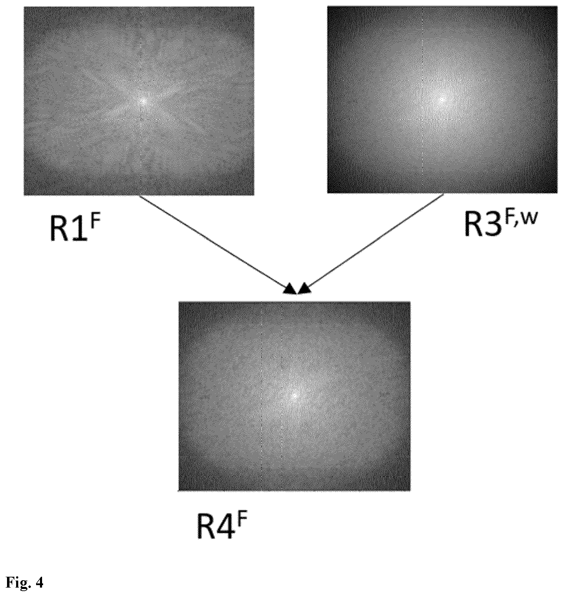

Fig. 4 zeigt beispielhaft und schematisch die Erzeugung einer vierten Repräsentation. In Fig. 4 wird eine vierte Repräsentation $R4^F$ des Untersuchungsbereichs des Untersuchungsobjekts im Frequenzraum durch die Kombination der bereits in Fig. 1 gezeigten ersten Frequenzraum-Repräsentation $R1^F$ mit der bereits in Fig. 2 gezeigten gewichteten Repräsentation $R3^{F,w}$ erzeugt. Die Kombination erfolgt in dem Beispiel durch Addition. Es ist möglich, die vierte Repräsentation $R4^F$ einer Normalisierung zu unterziehen.

In einem weiteren Schritt wird die vierte Frequenzraum-Repräsentation mittels einer Transformation (z.B. einer inversen Fourier-Transformation) in eine Ortraum-Repräsentation umgewandelt. Die Ortsraumdarstellung des Untersuchungsbereichs kann ausgegeben werden (z.B. auf einem Bildschirm angezeigt oder mit Hilfe eines Druckers ausgedruckt werden), auf einem Datenspeicher gespeichert werden und/oder an ein separates Computersystem übermittelt werden.

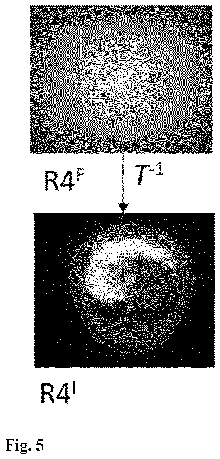

Fig. 5 zeigt beispielhaft und schematisch, wie aus der bereits in Fig. 4 gezeigten vierten Repräsentation $R4^F$ des Untersuchungsbereichs im Frequenzraum mittels der in Bezug zu Fig. 1 beschriebenen inversen Trans-

formation T$^{-1}$ eine vierte Repräsentation R4$^{I}$ des Untersuchungsbereichs im Ortsraum erzeugt wird.

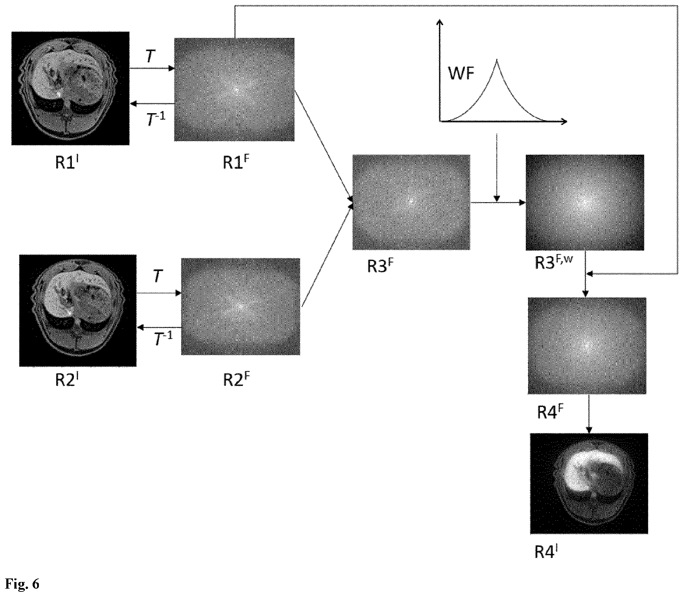

Fig. 6 zeigt beispielhaft und schematisch den gesamten Prozess, wie er anteilig bereits in Fig. 1, Fig. 2, Fig. 4 und Fig. 5 gezeigt ist.

Für den Fall, dass die dritte Repräsentation durch Subtraktion der ersten Repräsentation von der zweiten Repräsentation erzeugt wird, würde eine Addition der (nicht gewichteten) dritten Repräsentation zu der ersten Repräsentation wieder die zweite Repräsentation ergeben.

Eine Addition der gewichteten dritten Repräsentation zu der ersten Repräsentation ergibt eine andere Repräsentation als die zweite Repräsentation.

Durch die Gewichtung werden verschiedene Punkte erreicht: zum einen wird ein Fokus auf die Kontrastinformationen gelegt, d.h., es werden Merkmale in der resultierenden vierten Repräsentation hervorgehoben, die auf eine Kontraststeigerung durch die zweite Menge an Kontrastmittel zurückzuführen sind; zum anderen ist es möglich, die gewichtete dritte Repräsentation mehrfach zu der dritten Repräsentation zu addieren, um eine weitere Kontrasterhöhung zu erreichen, ohne dass Störungen und/oder Rauschen im selben Maße verstärkt werden wie der Kontrast.

Es ist also möglich, die gewichtete dritte Repräsentation multipliziert mit einem Verstärkungsfaktor zu der ersten Repräsentation zu addieren, wobei der Verstärkungsfaktor angibt, in welchem Maß der Kontrast in der vierten Repräsentation erhöht wird. Dabei ist es auch möglich, eine Verstärkung zu wählen, die kleiner als 1 ist, d.h. der Kontrast zwischen Bereichen mit Kontrastmittel und Bereichen ohne Kontrastmittel ist in der vierten Repräsentation (bzw. der korrespondierenden Ortsraumdarstellung) geringer als in der zweiten Repräsentation. Ebenso ist es möglich, eine Verstärkung zu erzielen, die größer ist als die durch eine Standardmenge an Kontrastmittel hervorgerufene Kontrastverstärkung. Eine solche Kontrastverstärkung ist mit dem in WO2019/074938A1 beschriebenen Verfahren nicht möglich, ohne dass man zur Erzeugung der Trainingsdaten Personen eine Menge an Kontrastmittel appliziert, die höher als die Standardmenge ist und damit außerhalb des von der Zulassungsbehörde zugelassenen Bereichs liegt.

Der Verstärkungsfaktor kann von einem Nutzer gewählt werden, d.h. variabel sein, oder vordefiniert sein, d.h. vorgegeben sein.

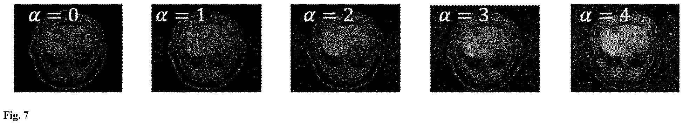

Fig. 7 zeigt beispielhaft und schematisch verschiedene Repräsentationen eines Untersuchungsbereichs eines Untersuchungsobjekts im Ortsraum. Die Repräsentationen unterscheiden sich durch den Verstärkungsfaktor $\alpha$, der in den vorliegenden Beispielen die Werte 0, 1, 2, 3 und 4 annehmen kann.

Ein Verstärkungsfaktor von $\alpha$ = 0 bedeutet, dass bei der ersten Repräsentation keine Kontrastverstärkung vorgenommen wird. Die Repräsentation zeigt also die ursprüngliche erste Repräsentation im Ortsraum.

Ein Verstärkungsfaktor von $\alpha$ = 1 bedeutet, dass zu der ersten Repräsentation im Frequenzraum die gewichtete dritte Frequenzraum-Repräsentation einmal addiert wird. Die Kontrastverstärkung ist ähnlich zu der Kontrastverstärkung der zweiten Ortsraum-Repräsentation, weist aber aufgrund der vorgenommenen stärkeren Gewichtung der niedrigen Frequenzen weniger Rauschen/Störungen auf.

Ein Verstärkungsfaktor von $\alpha$ = 2, 3 oder 4 bedeutet, dass zu der ersten Repräsentation im Frequenzraum die gewichtete dritte Frequenzraum-Repräsentation 2-mal, 3-mal oder 4-mal addiert wird. Die Kontrastverstärkung steigt mit zunehmendem Verstärkungsfaktor an.

In dem in Fig. 7 gezeigten Beispiel wird stets ein ganzzahliges Vielfaches der gewichteten dritten Frequenzraum-Repräsentation zu der ersten Repräsentation addiert. Natürlich ist es auch möglich, einen nichtganzzahligen Anteil der gewichteten dritten Frequenzraum-Repräsentation zu der ersten Repräsentation zu addieren (z.B. $\alpha$ = 1,5 oder $\alpha$ = 3,7 oder $\alpha$ = 4,159).

Es sind auch negative Werte von $\alpha$ möglich, die z.B. so gewählt werden können, dass Bereiche des Untersuchungsbereichs, die eine Kontrastmittel-induzierte Signal-Verstärkung in den messtechnisch erzeugten Repräsentation erfahren, in den künstlich erzeugten Repräsentationen komplett dunkel (schwarz) sind.

Es ist auch möglich, den Verstärkungsfaktor automatisch zu ermitteln. Es ist z.B. möglich, in einer Ortsraumdarstellung der ersten und/oder der zweiten Repräsentation mindestens einen Bereich zu definieren und/oder von einem Nutzer auswählen zu lassen und den Verstärkungsfaktor so einzustellen, dass ein Grauwert in der Ortsraumdarstellung (oder ein anderer Tonwert im Falle einer anderen Darstellung als einer Grauwertdarstellung) einen definierten Wert annimmt und/oder oberhalb oder unterhalb eines Schwellenwerts liegt und/oder zwei Grauwerte in zwei verschiedenen ausgewählten oder definierten Bereichen einen definierten Abstand voneinander haben und/oder einen Abstand voneinander aufweisen, der oberhalb oder unterhalb eines Schwellenwerts liegt. Es ist auch möglich, andere Kriterien anzuwenden, um den Verstärkungsfaktor automatisch zu ermitteln. Grundlage für die Kriterien zur automatischen Ermittlung des Verstärkungsfaktor kann beispielsweise das Histogramm der in eine Ortsraumdarstellung transformierten ersten, zweiten, dritten, gewichteten dritten und/oder vierte Repräsentation sein. In einem solchen Histogramm können die Zahl der Bildelemente mit einem definierten Tonwert oder Grauwert aufgeführt sein.

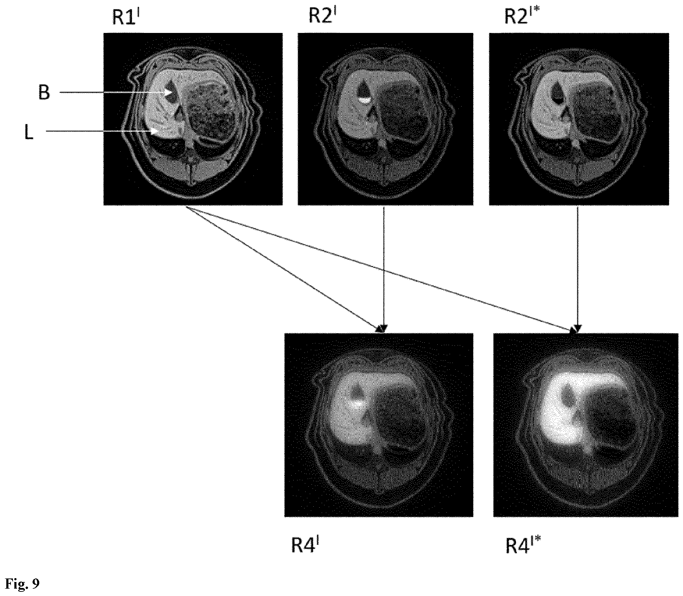

Fig. 9 zeigt eine bevorzugte Ausführungsform einer Ausgabe der künstlichen kontrastverstärkten radiologischen Aufnahme eines Untersuchungsbereichs mittels eines Computersystems/Computerprogramms. Die Ausgabe erfolgt gegenüber einem Nutzer des Computersystems und/oder Computerprogramms der vorliegenden Offenbarung.

Dem Nutzer werden eine erste Repräsentation R1$^l$ eines Untersuchungsbereichs eines Untersuchungsobjekts, eine zweite Repräsentation R2$^l$ des Untersuchungsbereichs des Untersuchungsobjekts und eine vorhersagte vierte Repräsentation R4$^l$ des Untersuchungsbereichs des Untersuchungsobjekts angezeigt (zum Beispiel auf einem Monitor).

Die erste Repräsentation R1$^l$ repräsentiert den Untersuchungsbereich ohne Kontrastmittel oder nach der Applikation einer ersten Menge eines Kontrastmittels.

Die zweite Repräsentation R2$^l$ repräsentiert den Untersuchungsbereich nach der Applikation einer zweiten Menge des Kontrastmittels. Die zweite Menge ist größer als die erste Menge.

Die vierte Repräsentation R4$^l$ repräsentiert den Untersuchungsbereich mit einem verstärkten Kontrast. Der Kontrast zwischen Bereichen ohne Kontrastmittel und Bereichen mit Kontrastmittel ist im Fall der vierten Repräsentation R4$^l$ größer als bei der zweiten Repräsentation R2$^l$.

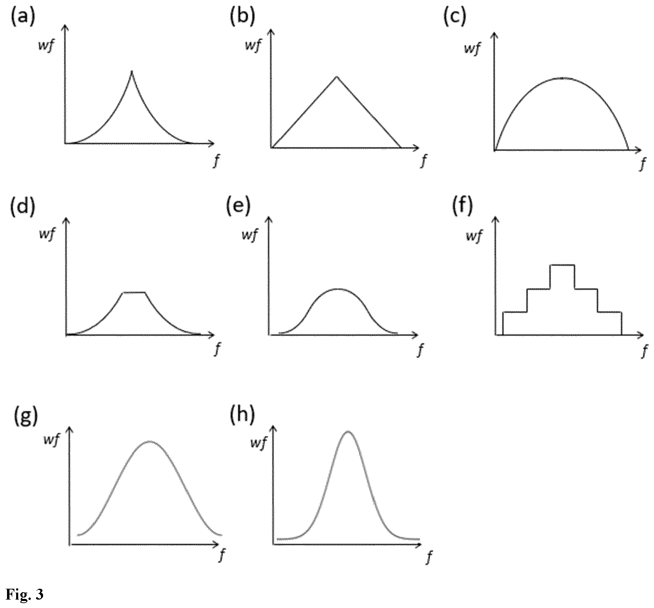

Die vierte Repräsentation R4$^l$ wurde wie in dieser Offenbarung beschrieben (siehe insbesondere die Beschreibungen zu Fig. 1 bis Fig. 7) erzeugt.

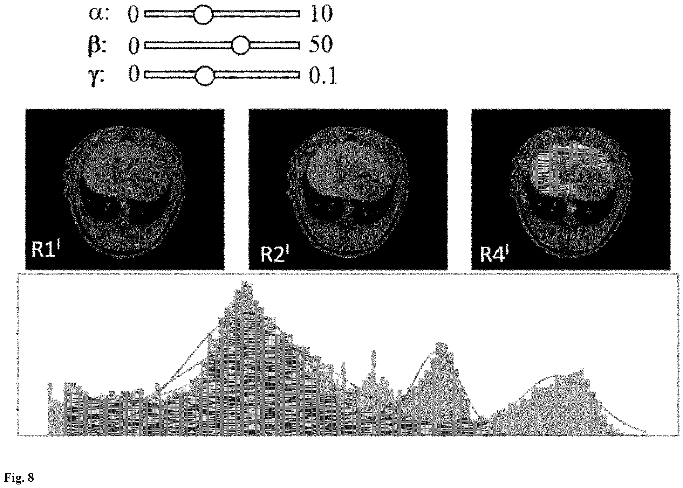

Alle angezeigten Repräsentationen sind Repräsentationen des Untersuchungsbereichs im Ortsraum. In dem in Fig. 8 gezeigten Beispiel werden dem Nutzer keine Frequenzraum-Repräsentationen angezeigt. Dies ist üblicherweise auch nicht vorgesehen, da viele Nutzer mit Frequenzraum-Repräsentation nicht vertraut sind.

Unterhalb der angezeigten Repräsentationen R1$^l$, R2$^l$ und R4$^l$ werden dem Nutzer die Histogramme der Repräsentationen in einer überlagerten Darstellung angezeigt.

Oberhalb der angezeigten Repräsentationen R1$^l$, R2$^l$ und R4$^l$ werden dem Nutzer drei virtuelle Schieberegler zur Verfügung gestellt, mit denen der Nutzer Einstellungen vornehmen kann. Mit einem ersten Schieberegler $\alpha$ kann der Nutzer den Verstärkungsfaktor im Bereich von 1 bis 10 wählen.

Mit einem zweiten Schieberegler $\beta$ und einem dritten Schieberegler $\gamma$ kann der Nutzer Parameter der Gewichtungsfunktion ändern. Diese Parameter können beispielsweise bestimmen, wie stark die Gewichtsfaktoren mit zunehmender Frequenz abnehmen.

Die in Fig. 8 gezeigte Ausgabe ist vorzugsweise so konfiguriert, dass die Anzeige der vierten Repräsentation R4$^l$ unmittelbar aktualisiert wird, wenn der Nutzer Änderungen mittels eines der Schieberegler vornimmt. Der Nutzer kann dann beispielsweise den Verstärkungsfaktor $\alpha$ ändern und unmittelbar sehen, wie sich eine Änderung des Verstärkungsfaktors auf die Repräsentation R4$^l$ auswirkt.

So kann er diejenigen Einstellungen identifizieren, die zu einer für den Nutzer optimalen vierten Repräsentation R4$^l$ des Untersuchungsbereichs führen.

Jede Änderung eines der Parameter $\alpha$, $\beta$ und/oder $\gamma$ führt bei dem Computersystem dazu, dass auf Basis des/der geänderten Parameter(s) die vierte Repräsentation R4$^l$ jeweils nur im Hintergrund berechnet und angezeigt wird. Dies gilt analog auch für das Histogramm der vierte Repräsentation R4$^l$.

Es ist möglich, dass durch die Kontrastverstärkung gemäß der bisherigen vorliegenden Offenbarung eine von einem Nutzer unerwünschte Kontrastverstärkung hervorgerufen wird. Dies sei an einem Beispiel erläutert. Das Beispiel ist schematisch in Fig. 9 gezeigt.

Fig. 9 zeigt eine erste Repräsentation R1$^l$ und eine zweite Repräsentation R2$^l$ eines Untersuchungsbereichs eines Untersuchungsobjekts. Der Untersuchungsbereich umfasst die Leber L und die Gallenblase B eines Schweines. Die erste Repräsentation R1$^l$ repräsentiert den Untersuchungsbereich ohne Kontrastmittel oder nach der Applikation einer ersten Menge eines Kontrastmittels im Ortsraum. Die zweite Repräsentation R2$^l$ repräsentiert den Untersuchungsbereich nach der Applikation einer zweiten Menge des Kontrastmittels im Ortsraum. Die zweite Menge ist größer als die erste Menge. In der zweiten Repräsentation R2$^l$ ist zu erkennen, dass sich die Gallenblase teilweise gefüllt hat, beispielsweise mit einer Flüssigkeit umfassend das Kontrastmittel oder einer anderen Flüssigkeit, die zu einem hohen Kontrast zwischen der teilweise gefüllten Gallenblase und den umliegenden Bereichen führt.

Eine Kontrastverstärkung wie in der bisherigen Offenbarung beschrieben führt dazu, dass in einer künstlichen kontrastverstärkten radiologischen Aufnahme R4$^l$ des Untersuchungsbereichs, der Kontrast zwischen der teilgefüllten Gallenblase und den anderen Bereichen noch verstärkt wird. Es ist aber denkbar, dass ein Nutzer eher an einer Kontrastverstärkung der Leber interessiert ist.

In einer bevorzugten Ausführungsform sind das Computersystem und das Computerprogramm der vorliegenden Offenbarung konfiguriert, von dem Nutzer eine Eingabe zu empfangen. In der Eingabe kann der Nutzer einen oder mehrere Bereiche spezifizieren, für die er keine Kontrastverstärkung wünscht. Der Nutzer kann einen solchen Bereich beispielsweise mittels einer Maus oder einem anderen Eingabemittel in der ersten, zweiten und/oder vierten Repräsentation im Ortsraum einzeichnen. So kann der Nutzer in dem in Fig. 9 gezeigten Beispiel die Gallenblase in der ersten Repräsentation R1$^l$, der zweiten Repräsentation R2$^l$ und/oder der vierten Repräsentation R4$^l$ auswählen und/oder markieren. Das Computersystem und das Computerprogramm können konfiguriert sein, die Tonwerte oder Grauwerte aller Bildelemente (Pixel, Voxel) die die (markiert) Gallenblase repräsentieren, auf Null zu setzen. Das Ergebnis ist die Repräsentation R2$^{l*}$, in der die Gallenblase durch schwarze Bildelemente repräsentiert wird. Führt man die Kontrastverstärkung wie in dieser Offenbarung beschrieben auf Basis der Repräsentationen R1$^l$ und R2$^{l*}$ (bzw. auf Basis ihrer korrespondierenden Frequenzraum-Repräsentationen) durch, ergibt sich die künstliche kontrastverstärkte radiologische Aufnahme R4$^{I*}$, in der nun insbesondere der Kontrast zwischen der Leber L und den übrigen Bereichen verstärkt ist, aber die teilweise gefüllte Gallenblase nicht mehr kontrastverstärkt dargestellt ist.

In einer bevorzugten Ausführungsform werden Bereiche, die nicht kontrastverstärkt werden (sollen), automatisch ermittelt. Vorzugsweise wird für alle paarweise korrespondierenden Bildelemente der ersten Ortsraum-Repräsentation R1$^{I}$ und der zweiten Ortsraum-Repräsentation R2$^{I}$ der Quotient der Tonwerte ermittelt:

$$Q = g2(x, y, z) \, / \, g1(x, y, z)$$

Dabei ist Q ein Quotient von Tonwerten, $g2(x, y, z)$ ist der Tonwert des Bildelements mit den Koordinaten x, y, z in der zweiten Repräsentation R2$^{I}$ und $g1(x, y, z)$ ist der Tonwert des Bildelements mit denselben Koordinaten x, y, z in der ersten Repräsentation R1$^{I}$. Der Quotient Q der Tonwerte ist ein Maß dafür, wieviel heller das Bildelement mit den Koordinaten x, y, z in der zweiten Repräsentation gegenüber dem korrespondierenden Bildelement in der ersten Repräsentation dargestellt wird. Er gibt die Kontrastverstärkung an, die durch die zweite Menge des Kontrastmittels in dem Untersuchungsbereich, der durch Bildelement mit den Koordinaten x, y, z repräsentiert wird, verursacht wird.

Das Computersystem und das Computerprogramm können konfiguriert sein, den Quotienten der Tonwerte für alle Bildelemente mit einem vordefinierten Schwellenwert zu vergleichen. Der vordefinierte Schwellenwert gibt eine maximale durch das Kontrastmittel zu erwartende Kontrastverstärkung an.

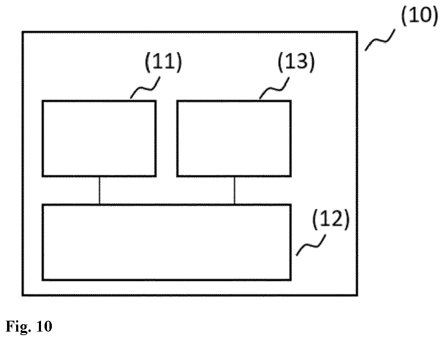

Ist ein Quotient der Tonwerte korrespondierender Bildelemente größer als der vordefinierte Schwellenwert, so können die Tonwerte der korrespondierenden Bildelemente auf Null gesetzt werden. Fig. 10 zeigt beispielhaft und schematisch ein Computersystem gemäß der vorliegenden Offenbarung.

Ein "Computersystem" ist ein System zur elektronischen Datenverarbeitung, das mittels programmierbarer Rechenvorschriften Daten verarbeitet. Ein solches System umfasst üblicherweise einen "Computer", diejenige Einheit, die einen Prozessor zur Durchführung logischer Operationen umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Laufwerke, Kamera, Mikrofon, Lautsprecher etc. Auch interne Anschlüsse und Erweiterungskarten gelten in der Computertechnik als Peripherie.

Das in Fig. 10 gezeigte Computersystem (1) umfasst eine Eingabeeinheit (10), eine Steuer- und Recheneinheit (20) und eine Ausgabeeinheit (30).

Die Steuer- und Recheneinheit (20) dient der Steuerung des Computersystems (1), der Koordinierung der Datenflüsse zwischen den Einheiten des Computersystems (1) und der Durchführung von Berechnungen.

Die Steuer- und Recheneinheit (20) ist konfiguriert:

- die Empfangseinheit zu veranlassen, eine erste Repräsentation zu empfangen, wobei die erste Repräsentation einen Untersuchungsbereich eines Untersuchungsobjekts ohne Kontrastmittel oder nach der Applikation einer ersten Menge eines Kontrastmittels im Frequenzraum repräsentiert,
- die Empfangseinheit zu veranlassen, eine zweite Repräsentation zu empfangen, wobei die zweite Repräsentation den Untersuchungsbereich des Untersuchungsobjekts nach einer Applikation einer zweiten Menge des Kontrastmittels im Frequenzraum repräsentiert,
- eine dritte Repräsentation auf Basis der ersten Repräsentation und der zweiten Repräsentation zu erzeugen, wobei die dritte Repräsentation die im Untersuchungsbereich durch die zweite Menge an Kontrastmittel hervorgerufene Signalverstärkung im Frequenzraum repräsentiert,
- eine gewichtete dritte Repräsentation durch Anwenden einer frequenzabhängigen Gewichtsfunktion auf die dritte Repräsentation zu erzeugen,
- eine vierte Repräsentation durch Kombinieren der ersten Repräsentation mit der gewichteten dritten Repräsentation zu erzeugen,
- die vierte Repräsentation in eine Repräsentation des Untersuchungsbereichs im Ortsraum zu transformieren,
- die Ausgabeeinheit zu veranlassen, die Repräsentation des Untersuchungsbereichs im Ortsraum auszugeben und/oder zu speichern und/oder an ein separates Computersystem zu übermitteln.

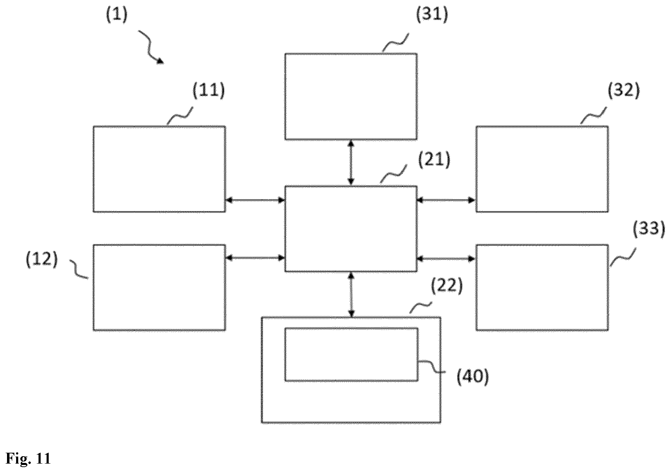

Fig. 11 zeigt beispielhaft und schematisch eine weitere Ausführungsform des Computersystems.

Das Computersystem (1) umfasst eine Verarbeitungseinheit (21), die mit einem Speicher (22) verbunden ist. Die Verarbeitungseinheit (21) und der Speicher (22) bilden eine Steuer- und Berechnungseinheit, wie sie in Fig. 10 gezeigt ist.

Die Verarbeitungseinheit (21) (engl.: *processing unit*) kann einen oder mehrere Prozessoren allein oder in Kombination mit einem oder mehreren Speichern umfassen. Bei der Verarbeitungseinheit (21) kann es sich um gewöhnliche Computerhardware handeln, die in der Lage ist, Informationen wie z.B. digitale Bildaufnahmen, Computerprogramme und/oder andere digitale Informationen zu verarbeiten. Die Verarbeitungseinheit (21) besteht üblicherweise aus einer Anordnung elektronischer Schaltungen, von denen einige als integrierter Schaltkreis oder als mehrere miteinander verbundene integrierte Schaltkreise (ein integrierter Schaltkreis wird manchmal auch als "Chip" bezeichnet) ausgeführt sein können.

Die Verarbeitungseinheit (21) kann konfiguriert sein, Computerprogramme auszuführen, die in einem Arbeitsspeicher der Verarbeitungseinheit (21) oder im Speicher (22) desselben oder eines anderen Computersystems gespeichert sein können.

Der Speicher (22) kann eine gewöhnliche Computerhardware sein, die in der Lage ist, Informationen wie z.B. digitale Bildaufnahmen (z.B. Repräsentationen des Untersuchungsbereichs), Daten, Computerprogramme und/oder andere digitale Informationen entweder vorübergehend und/oder dauerhaft zu speichern. Der Speicher (22) kann einen flüchtigen und/oder nichtflüchtigen Speicher umfassen und kann fest eingebaut oder entfernbar sein. Beispiele für geeignete Speicher sind RAM (Random Access Memory), ROM (Read-Only Memory), eine Festplatte, ein Flash-Speicher, eine austauschbare Computerdiskette, eine optische Disc, ein Magnetband oder eine Kombination der oben genannten. Zu den optischen Discs können Compact Discs mit Nur-Lese-Speicher (CD-ROM), Compact Discs mit Lese-/Schreibfunktion (CD-R/W), DVDs, Blu-ray-Discs und ähnliche gehören.

Zusätzlich zum Speicher (22) kann die Verarbeitungseinheit (21) auch mit einer oder mehreren Schnittstellen (11, 12, 31, 32, 33) verbunden sein, um Informationen anzuzeigen, zu übertragen und/oder zu empfangen. Die Schnittstellen können eine oder mehrere Kommunikationsschnittstellen (32, 33) und/oder eine oder mehrere Benutzerschnittstellen (11, 12, 31) umfassen. Die eine oder mehrere Kommunikationsschnittstellen können so konfiguriert sein, dass sie Informationen senden und/oder empfangen, z.B. zu und/oder von einer MRT-Scanner, einem CT-Scanner, einer Ultraschallkamera, anderen Computersystemen, Netzwerken, Datenspeichern oder dergleichen. Die eine oder mehrere Kommunikationsschnittstellen können so konfiguriert sein, dass sie Informationen über physische (verdrahtete) und/oder drahtlose Kommunikationsverbindungen übertragen und/oder empfangen. Die eine oder die mehreren Kommunikationsschnittstellen können eine oder mehrere Schnittstellen für die Verbindung mit einem Netzwerk enthalten, z.B. unter Verwendung von Technologien wie Mobiltelefon, Wi-Fi, Satellit, Kabel, DSL, Glasfaser und/oder dergleichen. In einigen Beispielen können die eine oder die mehreren Kommunikationsschnittstellen eine oder mehrere Nahbereichskommunikationsschnittstellen umfassen, die so konfiguriert sind, dass sie Geräte mit Nahbereichskommunikationstechnologien wie NFC, RFID, Bluetooth, Bluetooth LE, ZigBee, Infrarot (z. B. IrDA) oder Ähnlichem verbinden.

Die Benutzerschnittstellen können eine Anzeige (31) umfassen. Eine Anzeige (31) kann so konfiguriert sein, dass sie einem Benutzer Informationen anzeigt. Geeignete Beispiele hierfür sind eine Flüssigkristallanzeige (LCD), eine Leuchtdiodenanzeige (LED), ein Plasmabildschirm (PDP) oder Ähnliches. Die Benutzereingabeschnittstelle(n) (11, 12) kann/können verdrahtet oder drahtlos sein und kann/können so konfiguriert sein, dass sie Informationen von einem Benutzer in das Computersystem (1) empfängt/empfangen, z.B. zur Verarbeitung, Speicherung und/oder Anzeige. Geeignete Beispiele für Benutzereingabeschnittstellen sind ein Mikrofon, ein Bild- oder Videoaufnahmegerät (z.B. eine Kamera), eine Tastatur oder ein Tastenfeld, ein Joystick, eine berührungsempfindliche Oberfläche (getrennt von einem Touchscreen oder darin integriert) oder ähnliches. In einigen Beispielen können die Benutzerschnittstellen eine automatische Identifikations- und Datenerfassungstechnologie (AIDC) für maschinenlesbare Informationen enthalten. Dazu können Barcodes, Radiofrequenz-Identifikation (RFID), Magnetstreifen, optische Zeichenerkennung (OCR), Karten mit integrierten Schaltkreisen (ICC) und ähnliches gehören. Die Benutzerschnittstellen können ferner eine oder mehrere Schnittstellen für die Kommunikation mit Peripheriegeräten wie Druckern und dergleichen umfassen.

Ein oder mehrere Computerprogramme (40) können im Speicher (22) gespeichert sein und von der Verarbeitungseinheit (21) ausgeführt werden, die dadurch programmiert wird, die in dieser Beschreibung beschriebenen Funktionen zu erfüllen. Das Abrufen, Laden und Ausführen von Anweisungen des Computerprogramms (40) kann sequenziell erfolgen, so dass jeweils ein Befehl abgerufen, geladen und ausgeführt wird. Das Abrufen, Laden und/oder Ausführen kann aber auch parallel erfolgen.

Das erfindungsgemäße System kann als Laptop, Notebook, Netbook und/der Tablet-PC ausgeführt sein, es kann auch ein Bestandteil eines MRT-Scanners, eines CT-Scanners oder eines Ultraschalldiagnosegeräts sein.

## Patentansprüche

1. Computer-implementiertes Verfahren umfassend die Schritte:

   - Empfangen oder Erzeugen einer ersten Repräsentation ($R1^F$), wobei die erste Repräsentation ($R1^F$) einen Untersuchungsbereich eines Untersuchungsobjekts ohne Kontrastmittel oder nach der Applikation einer ersten Menge eines Kontrastmittels im Frequenzraum repräsentiert,
   - Empfangen oder Erzeugen einer zweiten Repräsentation ($R2^F$), wobei die zweite Repräsentation ($R2^F$) den Untersuchungsbereich des Untersuchungsobjekts nach einer Applikation einer zweiten Menge des Kontrastmittels im Frequenzraum repräsentiert,
   - Erzeugen einer dritten Repräsentation ($R3^F$) auf Basis der ersten Repräsentation ($R1^F$) und der zweiten Repräsentation ($R2^F$), wobei die dritte Repräsentation ($R3^F$) die im Untersuchungsbereich durch die zweite Menge an Kontrastmittel hervorgerufene Signalverstärkung im Frequenzraum repräsentiert,
- Erzeugen einer gewichteten dritten Repräsentation ($R3^{F,w}$) durch Anwenden einer frequenzabhängigen Gewichtsfunktion (WF) auf die dritte Repräsentation ($R3^F$),
- Erzeugen einer vierten Repräsentation ($R4^F$) durch Kombinieren der ersten Repräsentation ($R1^F$) mit der gewichteten dritten Repräsentation ($R3^{F,w}$),
- Transformieren der vierten Repräsentation ($R4^F$) in eine Repräsentation ($R4^I$) des Untersuchungsbereichs im Ortsraum,
- Ausgeben und/oder Speichern der Repräsentation ($R4^I$) des Untersuchungsbereichs im Ortsraum und/oder Übermitteln der Repräsentation ($R4^I$) des Untersuchungsbereichs im Ortsraum an ein separates Computersystem.

2. Verfahren nach Anspruch 1, wobei das Untersuchungsobjekt ein Lebewesen, vorzugsweise ein Säugetier, ganz besonders bevorzugt ein Mensch ist.

3. Verfahren nach Anspruch 1 oder 2, wobei der Untersuchungsbereich eine Leber, eine Niere, ein Herz, eine Lunge, ein Gehirn, ein Magen, eine Blase, eine Prostatadrüse, ein Darm oder ein Teil davon oder ein anderer Teil des Körpers eines Menschen ist.

4. Verfahren nach einem der Ansprüche 1 bis 3, umfassend:

   - Empfangen einer ersten Ortsraum-Repräsentation ($R1^I$), wobei die erste Ortsraum-Repräsentation ($R1^I$) den Untersuchungsbereich des Untersuchungsobjekts ohne Kontrastmittel oder nach der Applikation der ersten Menge des Kontrastmittels im Ortsraum repräsentiert,
   - Transformieren der ersten Ortsraum-Repräsentation ($R1^I$) in die erste Repräsentation ($R1^F$) des Untersuchungsbereich des Untersuchungsobjekts im Frequenzraum,
   - Empfangen einer zweiten Ortsraum-Repräsentation ($R2^I$), wobei die zweite Ortsraum-Repräsentation ($R2^I$) den Untersuchungsbereich des Untersuchungsobjekts nach der Applikation der zweiten Menge des Kontrastmittels im Ortsraum repräsentiert,
   - Transformieren der zweiten Ortsraum-Repräsentation ($R2^I$) in die zweite Repräsentation ($R2^F$) des Untersuchungsbereich des Untersuchungsobjekts im Frequenzraum.

5. Verfahren nach einem der Ansprüche 1 bis 4, wobei das Erzeugen der dritten Repräsentation ($R3^F$) auf Basis der ersten Repräsentation ($R1^F$) und der zweiten Repräsentation ($R2^F$) umfasst: Subtrahieren der ersten Repräsentation ($R1^F$) von der zweiten Repräsentation ($R2^F$).

6. Verfahren nach einem der Ansprüche 1 bis 5, wobei das Kombinieren der ersten Repräsentation ($R1^F$) mit der gewichteten dritten Repräsentation ($R3^{F,w}$) umfasst: Addieren der ersten Repräsentation ($R1^F$) und der gewichteten dritten Repräsentation ($R3^{F,w}$).

7. Verfahren nach einem der Ansprüche 1 bis 6, wobei durch das Anwenden der frequenzabhängigen Gewichtsfunktion (WF) auf die dritte Repräsentation ($R3^F$) Amplitudenwerte mit einer niedrigen Frequenz mit einem größeren Gewichtsfaktor multipliziert werden als Amplitudenwerte mit einer hohen Frequenz.

8. Verfahren nach einem der Ansprüche 1 bis 7, wobei beim Anwenden der frequenzabhängigen Gewichtsfunktion (WF) auf die dritte Repräsentation ($R3^F$) Amplitudenwerte mit einer Fensterfunktion multipliziert werden, wobei die Fensterfunktion eine Gauß-Verteilungsfunktion oder eine Hann-Funktion ist.

9. Verfahren nach einem der Ansprüche 1 bis 8, wobei das Kombinieren der ersten Repräsentation ($R1^F$) mit der gewichteten dritten Repräsentation ($R3^{F,w}$) umfasst: Addieren das $\alpha$-fache der gewichteten dritten Repräsentation ($R3^{F,w}$) zu der ersten Repräsentation ($R1^F$), wobei $\alpha$ eine negative oder positive reelle Zahl ist.

10. Verfahren nach Anspruch 9, ferner umfassend:

    - Empfangen eines oder mehrerer Werte für $\alpha$ von einem Nutzer.

11. Verfahren nach Anspruch 9, ferner umfassend:

    - Empfangen eines ersten Tonwerts eines ersten Bildelements einer Ortsraumdarstellung ($R1^I$) der ersten Repräsentation oder einer Ortsraumdarstellung ($R2^I$) der zweiten Repräsentation,
    - Empfangen eines zweiten Tonwerts eines zweiten Bildelements einer Ortsraumdarstellung ($R1^I$) der ersten Repräsentation oder einer Ortsraumdarstellung ($R2^I$) der zweiten Repräsentation,
    - Ermitteln eines Wertes für $\alpha$, für den die Differenz zwischen dem ersten Tonwert und dem zweiten Tonwert einen vordefinierten Wert annimmt oder oberhalb oder unterhalb eines vordefinierten Schwellenwerts liegt.

12. Verfahren nach einem der Ansprüche 1 bis 11, ferner umfassend:

    - Empfangen einer Markierung eines Bereichs innerhalb einer Ortsraum-Darstellung (R2$^I$) der zweiten Repräsentation,
- Setzen der Tonwerte des Bereichs auf Null,
- Erzeugen der zweiten Repräsentation (R2$^F$) aus der Ortsraum-Darstellung.

13. Verfahren nach einem der Ansprüche 1 bis 11, ferner umfassend:

- für alle Koordinaten einer ersten Ortraumdarstellung (R1$^I$) der ersten Repräsentation: Ermitteln eines ersten Tonwerts,
- für alle Koordinaten einer zweiten Ortraumdarstellung (R2$^I$) der zweiten Repräsentation: Ermitteln eines zweiten Tonwerte,
- für alle Koordinaten der ersten Ortraumdarstellung (R1$^I$) und der zweiten Ortraumdarstellung (R2$^I$): Ermitteln eines Quotienten aus zweitem Tonwert und erstem Tonwert,
- Setzten der Tonwerte der zweiten Ortsraumdarstellung (R2$^I$) auf Null, für die der Quotient größer als ein vordefinierter Schwellenwert ist,
- Erzeugen der zweiten Repräsentation (R2$^F$) aus der zweiten Ortsraumdarstellung (R2$^I$).

14. Computersystem (10) umfassend

• eine Empfangseinheit (11),
• eine Steuer- und Recheneinheit (12) und
• eine Ausgabeeinheit (13),
wobei die Steuer- und Recheneinheit (12) konfiguriert ist,

- die Empfangseinheit (11) zu veranlassen, eine erste Repräsentation (R1$^F$) zu empfangen, wobei die erste Repräsentation (R1$^F$) einen Untersuchungsbereich eines Untersuchungsobjekts ohne Kontrastmittel oder nach der Applikation einer ersten Menge eines Kontrastmittels im Frequenzraum repräsentiert, oder eine erste Ortsraum-Repräsentation (R1$^I$) zu empfangen, wobei die erste Ortsraum-Repräsentation (R1$^I$) den Untersuchungsbereich des Untersuchungsobjekts ohne Kontrastmittel oder nach der Applikation der ersten Menge des Kontrastmittels im Ortsraum repräsentiert,
- für den Fall, dass von der Empfangseinheit (11) die erste Ortsraum-Repräsentation (R1$^I$) empfangen wurde: die erste Ortsraum-Repräsentation (R1$^I$) in die erste Repräsentation (R1$^F$) des Untersuchungsbereich des Untersuchungsobjekts im Frequenzraum zu transformieren,
- die Empfangseinheit (11) zu veranlassen, eine zweite Repräsentation (R2$^F$) zu empfangen, wobei die zweite Repräsentation (R2$^F$) den Untersuchungsbereich des Untersuchungsobjekts nach einer Applikation einer zweiten Menge des Kontrastmittels im Frequenzraum repräsentiert, oder eine zweite Ortsraum-Repräsentation (R2$^I$) zu empfangen, wobei die zweite Ortsraum-Repräsentation (R2$^I$) den Untersuchungsbereich des Untersuchungsobjekts nach der Applikation der zweiten Menge des Kontrastmittels im Ortsraum repräsentiert,
- für den Fall, dass von der Empfangseinheit (11) die zweite Ortsraum-Repräsentation (R2$^I$) empfangen wurde: die zweite Ortsraum-Repräsentation (R2$^I$) in die zweite Repräsentation (R2$^F$) des Untersuchungsbereich des Untersuchungsobjekts im Frequenzraum zu transformieren,
- eine dritte Repräsentation (R3$^F$) auf Basis der ersten Repräsentation (R1$^F$) und der zweiten Repräsentation (R2$^F$) zu erzeugen, wobei die dritte Repräsentation (R3$^F$) die im Untersuchungsbereich durch die zweite Menge an Kontrastmittel hervorgerufene Signalverstärkung im Frequenzraum repräsentiert,
- eine gewichtete dritte Repräsentation (R3$^{F,w}$) durch Anwenden einer frequenzabhängigen Gewichtsfunktion (WF) auf die dritte Repräsentation (R3$^F$) zu erzeugen,
- eine vierte Repräsentation (R4$^F$) durch Kombinieren der ersten Repräsentation (R1$^F$) mit der gewichteten dritten Repräsentation (R3$^{F,w}$) zu erzeugen,
- die vierte Repräsentation (R4$^F$) in eine Repräsentation (R4$^I$) des Untersuchungsbereichs im Ortsraum zu transformieren,
- die Ausgabeeinheit (13) zu veranlassen, die Repräsentation (R4$^I$) des Untersuchungsbereichs im Ortsraum auszugeben und/oder zu speichern und/oder an ein separates Computersystem zu übermitteln.

15. Computerprogrammprodukt umfassend einen Datenträger, auf dem ein Computerprogramm (40) gespeichert ist, wobei das Computerprogramm (40) in einen Arbeitsspeicher (22) eines Computersystems (1) geladen werden kann und dort das Computersystem (1) dazu veranlasst, folgende Schritte ausführen:

- Empfangen oder Erzeugen einer ersten Repräsentation (R1$^F$), wobei die erste Repräsentation (R1$^F$) einen Untersuchungsbereich eines Untersuchungsobjekts ohne Kontrastmittel oder nach der Applikation einer ersten Menge eines Kontrastmittels im Frequenzraum repräsentiert,
- Empfangen oder Erzeugen einer zweiten Repräsentation (R2$^F$), wobei die zweite Repräsentation (R2$^F$) den Untersuchungsbereich des Untersuchungsobjekts nach einer Applikation einer zweiten Menge des Kontrastmittels im Frequenzraum repräsentiert,

- Erzeugen einer dritten Repräsentation (R3$^F$) auf Basis der ersten Repräsentation (R1$^F$) und der zweiten Repräsentation (R2$^F$), wobei die dritte Repräsentation (R3$^F$) die im Untersuchungsbereich durch die zweite Menge an Kontrastmittel hervorgerufene Signalverstärkung im Frequenzraum repräsentiert,
- Erzeugen einer gewichteten dritten Repräsentation (R3$^{F,w}$) durch Anwenden einer frequenzabhängigen Gewichtsfunktion (WF) auf die dritte Repräsentation (R3$^{F,w}$),
- Erzeugen einer vierten Repräsentation (R4$^F$) durch Kombinieren der ersten Repräsentation (R1$^F$) mit der gewichteten dritten Repräsentation (R3$^{F,w}$),
- Transformieren der vierten Repräsentation (R4$^F$) in eine Repräsentation (R4$^I$) des Untersuchungsbereichs im Ortsraum,
- Ausgeben und/oder Speichern der Repräsentation (R4$^I$) des Untersuchungsbereichs im Ortsraum und/oder Übermitteln der Repräsentation (R4$^I$) des Untersuchungsbereichs im Ortsraum an ein separates Computersystem.

16. Kit umfassend ein Computerprogrammprodukt gemäß Anspruch 15 und ein Kontrastmittel.

## Claims

1. Computer-implemented method, comprising the following steps:

   - receiving or generating a first representation (R1$^F$), wherein the first representation (R1$^F$) represents an examination region of an examination object without contrast agent or after application of a first amount of a contrast agent in frequency space,
   - receiving or generating a second representation (R2$^F$), wherein the second representation (R2$^F$) represents the examination region of the examination object after application of a second amount of the contrast agent in frequency space,
   - generating a third representation (R3$^F$) on the basis of the first representation (R1$^F$) and the second representation (R2$^F$), wherein the third representation (R3$^F$) represents the signal amplification in frequency space caused by the second amount of contrast agent in the examination region,
   - generating a weighted third representation (R3$^{F,w}$) by applying a frequency-dependent weight function (WF) to the third representation (R3$^F$),
   - generating a fourth representation (R4$^F$) by combining the first representation (R1$^F$) with the weighted third representation (R3$^{F,w}$),
   - transforming the fourth representation (R4$^F$) into a representation (R4$^I$) of the examination region in real space,
   - outputting and/or storing the representation (R4$^I$) of the examination region in real space and/or transmitting the representation (R4$^I$) of the examination region in real space to a separate computer system.

2. Method according to Claim 1, wherein the examination object is a living being, preferably a mammal, most preferably a human.

3. Method according to Claim 1 or 2, wherein the examination region is a liver, kidney, heart, lung, brain, stomach, bladder, prostate gland, intestine or a part thereof or another part of the body of a human.

4. Method according to one of Claims 1 to 3, comprising:

   - receiving a first real-space representation (R1$^I$), wherein the first real-space representation (R1$^I$) represents the examination region of the examination object without contrast agent or after application of the first amount of the contrast agent in real space,
   - transforming the first real-space representation (R1$^I$) into the first representation (R1$^F$) of the examination region of the examination object in frequency space,
   - receiving a second real-space representation (R2$^I$), wherein the second real-space representation (R2$^I$) represents the examination region of the examination object after application of the second amount of the contrast agent in real space,
   - transforming the second real-space representation (R2$^I$) into the second representation (R2$^F$) of the examination region of the examination object in frequency space.

5. Method according to one of Claims 1 to 4, wherein generating the third representation (R3$^F$) on the basis of the first representation (R1$^F$) and the second representation (R2$^F$) comprises: subtracting the first representation (R1$^F$) from the second representation (R2$^F$).

6. Method according to one of Claims 1 to 5, wherein combining the first representation (R1$^F$) with the weighted third representation (R3$^{F,w}$) comprises: adding the first representation (R1$^F$) and the weighted third representation (R3$^{F,w}$).

7. Method according to one of Claims 1 to 6, wherein, by applying the frequency-dependent weight function (WF) to the third representation (R3$^F$), amplitude values having a low frequency are multiplied by a larger weight factor than amplitude values having a high frequency.

8. Method according to one of Claims 1 to 7, wherein, when the frequency-dependent weight function (WF) is applied to the third representation (R3$^F$), amplitude values are multiplied by a window function, wherein the window function is a Gaussian distribution function or a Hann function.

9. Method according to one of Claims 1 to 8, wherein combining the first representation (R1$^F$) with the weighted third representation (R3$^{F,w}$) comprises: adding $\alpha$ times the weighted third representation (R3$^{F,w}$) to the first representation (R1$^F$), wherein $\alpha$ is a negative or positive real number.

10. Method according to Claim 9, furthermore comprising:

    - receiving one or more values of $\alpha$ from a user.

11. Method according to Claim 9, furthermore comprising:

    - receiving a first tonal value of a first image element of a real-space representation (R1$^I$) of the first representation or a real-space representation (R2$^I$) of the second representation,
    - receiving a second tonal value of a second image element of a real-space representation (R1$^I$) of the first representation or a real-space representation (R2$^I$) of the second representation,
    - determining a value of $\alpha$ for which the difference between the first tonal value and the second tonal value assumes a predefined value or is above or below a predefined threshold value.

12. Method according to one of Claims 1 to 11, furthermore comprising:

    - receiving a marker of a region within a real-space representation (R2$^I$) of the second representation,
    - setting the tonal values of the region to zero,
    - generating the second representation (R2$^F$) from the real-space representation.

13. Method according to one of Claims 1 to 11, furthermore comprising:

    - for all coordinates of a first real-space representation (R1$^I$) of the first representation: determining a first tonal value,
    - for all coordinates of a second real-space representation (R2$^I$) of the second representation: determining a second tonal value,
    - for all coordinates of the first real-space representation (R1$^I$) and of the second real-space representation (R2$^I$): determining a quotient of the second tonal value and the first tonal value,
    - setting the tonal values of the second real-space representation (R2$^I$) for which the quotient is greater than a predefined threshold value to zero,
    - generating the second representation (R2$^F$) from the second real-space representation (R2$^I$).

14. Computer system (10) comprising

    • a receiving unit (11),
    • a control and calculation unit (12) and
    • an output unit (13),
    wherein the control and calculation unit (12) is configured
    - to cause the receiving unit (11) to receive a first representation (R1$^F$), wherein the first representation (R1$^F$) represents an examination region of an examination object without contrast agent or after application of a first amount of a contrast agent in frequency space, or to receive a first real-space representation (R1$^I$), wherein the first real-space representation (R1$^I$) represents the examination region of the examination object without contrast agent or after application of the first amount of the contrast agent in real space,
    - in the event that the first real-space representation (R1$^I$) has been received by the receiving unit (11): to transform the first real-space representation (R1$^I$) into the first representation (R1$^F$) of the examination region of the examination object in frequency space,
    - to cause the receiving unit (11) to receive a second representation (R2$^F$), wherein the second representation (R2$^F$) represents the examination region of the examination object after application of a second amount of the contrast agent in frequency space, or to receive a second real-space representation (R2$^I$), wherein the second real-space representation (R2$^I$) represents the examination region of the examination object after application of the second amount of the contrast agent in real space,
    - in the event that the second real-space representation (R2$^I$) has been received by the receiving unit (11): to transform the second real-space representation (R2$^I$) into the second representation (R2$^F$) of the examination region of the examination object in frequency space,

- to generate a third representation (R3$^F$) on the basis of the first representation (R1$^F$) and the second representation (R2$^F$), wherein the third representation (R3$^F$) represents the signal amplification in frequency space caused by the second amount of contrast agent in the examination region,
- to generate a weighted third representation (R3$^{F,w}$) by applying a frequency-dependent weight function (WF) to the third representation (R3$^F$),
- to generate a fourth representation (R4$^F$) by combining the first representation (R1$^F$) with the weighted third representation (R3$^{F,w}$),
- to transform the fourth representation (R4$^F$) into a representation (R4$^I$) of the examination region in real space,
- to cause the output unit (13) to output the representation (R4$^I$) of the examination region in real space and/or to store it and/or to transmit it to a separate computer system.

15. Computer program product comprising a data carrier on which there is stored a computer program (40), wherein the computer program (40) is able to be loaded into a working memory (22) of a computer system (1), where it causes the computer system (1) to execute the following steps:

- receiving or generating a first representation (R1$^F$), wherein the first representation (R1$^F$) represents an examination region of an examination object without contrast agent or after application of a first amount of a contrast agent in frequency space,
- receiving or generating a second representation (R2$^F$), wherein the second representation (R2$^F$) represents the examination region of the examination object after application of a second amount of the contrast agent in frequency space,
- generating a third representation (R3$^F$) on the basis of the first representation (R1$^F$) and the second representation (R2$^F$), wherein the third representation (R3$^F$) represents the signal amplification in frequency space caused by the second amount of contrast agent in the examination region,
- generating a weighted third representation (R3$^{F,w}$) by applying a frequency-dependent weight function (WF) to the third representation (R3$^{F,w}$),
- generating a fourth representation (R4$^F$) by combining the first representation (R1$^F$) with the weighted third representation (R3$^{F,w}$),
- transforming the fourth representation (R4$^F$) into a representation (R4$^I$) of the examination region in real space,

- outputting and/or storing the representation (R4$^I$) of the examination region in real space and/or transmitting the representation (R4$^I$) of the examination region in real space to a separate computer system.

16. Kit comprising a computer program product according to Claim 15 and a contrast agent.

**Revendications**

1. Procédé mis en œuvre par ordinateur, comprenant les étapes suivantes consistant à :

- recevoir ou générer une première représentation (R1$^F$), la première représentation (R1$^F$) représentant une zone d'examen d'un objet à examiner sans agent de contraste ou après l'application d'une première quantité d'un agent de contraste dans l'espace fréquentiel,
- recevoir ou générer une deuxième représentation (R2$^F$), la deuxième représentation (R2$^F$) représentant la zone d'examen de l'objet à examiner après une application d'une deuxième quantité de l'agent de contraste dans l'espace fréquentiel,
- générer une troisième représentation (R3$^F$) sur la base de la première représentation (R1$^F$) et de la deuxième représentation (R2$^F$), dans lequel la troisième représentation (R3$^F$) représente l'amplification de signal dans l'espace fréquentiel provoquée par la deuxième quantité de l'agent de contraste,
- générer une troisième représentation pondérée (R3$^{F,W}$) en appliquant à la troisième représentation (R3$^F$) une fonction de pondération (WF) dépendant de la fréquence,
- générer une quatrième représentation (R4$^F$) en combinant la première représentation (R1$^F$) avec la troisième représentation pondérée (R3$^{F,W}$),
- transformer la quatrième représentation (R4$^F$) en une représentation (R4$^I$) de la zone d'examen dans l'espace local,
- sortir et/ou stocker la représentation (R4$^I$) de la zone d'examen dans l'espace local et/ou transmettre la représentation (R4$^I$) de la zone d'examen dans l'espace local à un système informatique séparé.

2. Procédé selon la revendication 1, dans lequel l'objet à examiner est un être vivant, de préférence un mammifère, de manière particulièrement préférée un être humain.

3. Procédé selon la revendication 1 ou 2, dans lequel la zone d'examen est un foie, un rein, un coeur, un poumon, un cerveau, un estomac, une vessie, une glande de prostate, un intestin ou une partie de celui-ci ou une autre partie du corps d'un être humain.

4. Procédé selon l'une quelconque des revendications 1 à 3, comprenant :

- la réception d'une première représentation d'espace local (R1$^I$), la première représentation d'espace local (R1$^I$) représentant la zone d'examen de l'objet à examiner sans agent de contraste ou après l'application de la première quantité de l'agent de contraste dans l'espace local,
- la transformation de la première représentation d'espace local (R1$^I$) en la première représentation (R1$^F$) de la zone d'examen de l'objet à examiner dans l'espace fréquentiel,
- la réception d'une deuxième représentation d'espace local (R2$^I$), la deuxième représentation d'espace local (R2$^I$) représentant la zone d'examen de l'objet à examiner après l'application de la deuxième quantité de l'agent de contraste dans l'espace local,
- la transformation de la deuxième représentation d'espace local (R2$^I$) en la deuxième représentation (R2$^F$) de la zone d'examen de l'objet à examiner dans l'espace fréquentiel.

5. Procédé selon l'une quelconque des revendications 1 à 4, dans lequel la génération de la troisième représentation (R3$^F$) sur la base de la première représentation (R1$^F$) et de la deuxième représentation (R2$^F$) comprend : la soustraction de la première représentation (R1$^F$) de la deuxième représentation (R2$^F$).

6. Procédé selon l'une quelconque des revendications 1 à 5, dans lequel la combinaison de la première représentation (R1$^F$) avec la troisième représentation pondérée (R3$^{F,W}$) comprend : l'addition de la première représentation (R1$^F$) et de la troisième représentation pondérée (R3$^{F,W}$).

7. Procédé selon l'une quelconque des revendications 1 à 6, dans lequel, en appliquant la fonction de pondération (WF) dépendant de la fréquence à la troisième représentation (R3$^F$), des valeurs d'amplitude d'une fréquence basse sont multipliées par un facteur de pondération supérieur à celui des valeurs d'amplitude d'une fréquence haute.

8. Procédé selon l'une quelconque des revendications 1 à 7, dans lequel, lors de l'application de la fonction de pondération (WF) dépendant de la fréquence à la troisième représentation (R3$^F$), des valeurs d'amplitude sont multipliées par une fonction de fenêtrage, la fonction de fenêtrage étant une fonction de distribution gaussienne ou une fonction de Hann.

9. Procédé selon l'une quelconque des revendications 1 à 8, dans lequel la combinaison de la première représentation (R1$^F$) avec la troisième représentation pondérée (R3$^{F,W}$) comprend : l'addition de $\alpha$ fois la troisième représentation pondérée (R3$^{F,W}$) à la première représentation (R1$^F$), $\alpha$ étant un nombre réel négatif ou positif.

10. Procédé selon la revendication 9, comprenant en outre :

- la réception d'une ou de plusieurs valeurs pour $\alpha$ de la part d'un utilisateur.

11. Procédé selon la revendication 9, comprenant en outre :

- la réception d'une première valeur tonale d'un premier pixel d'une représentation d'espace local (R1$^I$) de la première représentation ou d'une représentation d'espace local (R2$^I$) de la deuxième représentation,
- recevoir une deuxième valeur tonale d'un deuxième pixel d'une représentation d'espace local (R1$^I$) de la première représentation ou d'une représentation d'espace local (R2$^I$) de la deuxième représentation,
- établir une valeur de $\alpha$ pour laquelle la différence entre la première valeur tonale et la deuxième valeur tonale prend une valeur prédéfinie ou se situe au-dessus ou au-dessous d'une valeur seuil prédéfinie.

12. Procédé selon l'une quelconque des revendications 1 à 11, comprenant en outre :

- la réception d'un marquage d'une zone à l'intérieur d'une représentation d'espace local (R2$^I$) de la deuxième représentation,
- la mise à zéro des valeurs tonales de la zone,
- la génération de la deuxième représentation (R2$^F$) à partir de la représentation d'espace local.

13. Procédé selon l'une quelconque des revendications 1 à 11, comprenant en outre :

- pour toutes les coordonnées d'une première représentation d'espace local (R1$^I$) de la première représentation : l'établissement d'une première valeur tonale,
- pour toutes les coordonnées d'une deuxième représentation d'espace local (R2$^I$) de la deuxième représentation : l'établissement d'une deuxième valeur tonale,
- pour toutes les coordonnées de la première représentation d'espace local (R1$^I$) et de la deuxième représentation d'espace local (R2$^I$) : l'établissement d'un quotient de la deuxième valeur tonale et de la première valeur tonale,
- la mise à zéro des valeurs tonales de la deuxième représentation d'espace local (R2$^I$) pour lesquelles le quotient est supérieur à une valeur seuil prédéfinie,
- la génération de la deuxième représentation (R2$^F$) à partir de la deuxième représentation d'espace local (R2$^I$).

14. Système d'ordinateur (10) comprenant

• une unité de réception (11),
• une unité de commande et de calcul (12), et
• une unité de sortie (13),

l'unité de commande et de calcul (12) étant configurée pour

- amener l'unité réceptrice (11) à recevoir une première représentation (R1$^F$), la première représentation (R1$^F$) représentant une zone d'examen d'un objet à examiner sans agent de contraste ou après l'application d'une première quantité d'un agent de contraste dans l'espace fréquentiel, ou à recevoir une première représentation d'espace local (R1$^I$), la première représentation d'espace local (R1$^I$) représentant la zone d'examen de l'objet à examiner sans agent de contraste ou après l'application de la première quantité de l'agent de contraste dans l'espace local,
- dans le cas où l'unité réceptrice (11) a reçu la première représentation d'espace local (R1$^I$) : transformer la première représentation d'espace local (R1$^I$) en la première représentation (R1$^F$) de la zone d'examen de l'objet à examiner dans l'espace fréquentiel,
- amener l'unité réceptrice (11) à recevoir une deuxième représentation (R2$^F$), la deuxième représentation (R2$^F$) représentant la zone d'examen de l'objet à examiner après une application d'une deuxième quantité de l'agent de contraste dans l'espace fréquentiel, ou à recevoir une deuxième représentation d'espace local (R2$^I$), la deuxième représentation d'espace local (R2$^I$) représentant la zone d'examen de l'objet à examiner après l'application de la deuxième quantité de l'agent de contraste dans l'espace local,
- dans le cas où l'unité réceptrice (11) a reçu la deuxième représentation d'espace local (R2$^I$) : transformer la deuxième représentation d'espace local (R2$^I$) en la deuxième représentation (R2$^F$) de la zone d'examen de l'objet à examiner dans l'espace fréquentiel,
- générer une troisième représentation (R3$^F$) sur la base de la première représentation (R1$^F$) et de la deuxième représentation (R2$^F$), la troisième représentation (R3$^F$) représentant l'amplification de signal dans l'espace fréquentiel provoquée par la deuxième quantité d'agent de contraste,
- générer une troisième représentation pondérée (R3$^{F,W}$) en appliquant une fonction de pondération (WF) dépendant de la fréquence à la troisième représentation (R3$^F$),
- générer une quatrième représentation (R4$^F$) en combinant la première représentation (R1$^F$) avec la troisième représentation pondérée (R3$^{F,W}$),
- transformer la quatrième représentation (R4$^F$) en une représentation (R4$^I$) de la zone d'examen dans l'espace local,
- amener l'unité de sortie (13) à sortir et/ou à stocker la représentation (R4$^I$) de la zone d'examen dans l'espace local et/ou à la transmettre à un système informatique séparé.

15. Produit de programme informatique, comprenant un support de données sur lequel est stocké un programme informatique (40), le programme informatique (40) pouvant être chargé dans une mémoire vive (22) d'un système informatique (1) et y amenant le système informatique (1) à exécuter les étapes suivantes consistant à :

- recevoir ou générer une première représentation (R1$^F$), la première représentation (R1$^F$) représentant une zone d'examen d'un objet à examiner sans agent de contraste ou après l'application d'une première quantité d'un agent de contraste dans l'espace fréquentiel,
- recevoir ou générer une deuxième représentation (R2$^F$), la deuxième représentation (R2$^F$) représentant la zone d'examen de l'objet à examiner après une application d'une deuxième quantité de l'agent de contraste dans l'espace fréquentiel,
- générer une troisième représentation (R3$^F$) sur la base de la première représentation (R1$^F$) et de la deuxième représentation (R2$^F$), dans lequel la troisième représentation (R3$^F$) représente l'amplification de signal dans l'espace fréquentiel provoquée par la deuxième quantité de l'agent de contraste,
- générer une troisième représentation pondérée (R3$^{F,W}$) en appliquant à la troisième représentation (R3$^{F,W}$) une fonction de pondération (WF) dépendant de la fréquence,
- générer une quatrième représentation (R4$^F$) en combinant la première représentation (R1$^F$)

avec la troisième représentation pondérée $(R3^{F,W})$,

- transformer la quatrième représentation $(R4^F)$ en une représentation $(R4^I)$ de la zone d'examen dans l'espace local,

- sortir et/ou stocker la représentation $(R4^I)$ de la zone d'examen dans l'espace local et/ou transmettre la représentation $(R4^I)$ de la zone d'examen dans l'espace local à un système informatique séparé.

16. Kit, comprenant un produit de programme informatique selon la revendication 15 et un agent de contraste.

Figuren

Fig. 1

Fig. 2

(a) (b) (c) (d) (e) (f) (g) (h)

**Fig. 3**

R1$^F$

R3$^{F,w}$

R4$^F$

Fig. 4

$R4^F$    $T^{-1}$

$R4^I$

Fig. 5

Fig. 6

Fig. 7

Fig. 8

Fig. 9

**Fig. 10**

**Fig. 11**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2019074938 A1
- WO 2014033163 A1

- US 6039931 A

**In der Beschreibung aufgeführte Nicht-Patentliteratur**

- **A. S. L. JASCINTH et al.** Contrast Agents in computed tomography: A Review. *Journal of Applied Dental and Medical Sciences,* 2016, vol. 2 (2), 143-149
- **H. LUSIC et al.** X-ray-Computed Tomography Contrast Agents. *Chem. Rev.,* 2013, vol. 113 (3), 1641-1666, https://www.radiology.wisc.edu/wpcontent/uploads/2017/10/contrast-agents-tutorial.pdf
- **M. R. NOUGH et al.** Radiographie and magnetic resonances contrast agents: Essentials and tips for safe practices. *World J Radiol.,* 28. September 2017, vol. 9 (9), 339-349
- **L. C. ABONYI et al.** Intravascular Contrast Media in Radiography: Historical Development & Review of-Risk Factors for Adverse Reactions. *South American Journal of Clinical Research,* 2016, vol. 3 (1), 1-10

- *ACR Manual on Contrast Media,* 2020, ISBN 978-1-55903-012-0
- **A. IGNEE et al.** Ultrasound contrast agents. *Endosc Ultrasound,* November 2016, vol. 5 (6), 355-362
- **S. SKARE.** Rigid Body Image Realignment in Image Space vs. k-Space. *ISMRM SCIENTIFIC WORKSHOP on Motion Correction,* 2014, https://cds.ismrm.org/protected/Motion_14/Program/Syllabus/Skare.pdf
- **R. POHMANN et al.** Accurate phosphorus metabolite images of the human heart by 3D acquisition-weighted CSI. *Magnetic Resonance in Medicine: An Official Journal of the International Society for Magnetic Resonance in Medicine,* 2001, vol. 45 (5), 817-826